# EUROPEAN PATENT APPLICATION

(11) **EP 4 123 019 A1**
(43) Date of publication of application: **25.01.2023**
(21) Application number: 21772409.5
(22) Date of filing: 18.03.2021
(51) Int. Cl.: C12N 5/078, A61K 35/16, A61P 17/02, A61K 35/51, A61P 37/06, C12N 5/073, C12N 5/071, C12N 15/85, C12N 15/63

(54) **CORD BLOOD PLASMA-DERIVED EXOSOME OR MIMETIC THEREOF AND PHARMACEUTICAL USE THEREOF**

(30) Priority: 18.03.2020 KR 20200033254; 29.01.2021 KR 20210013569
(71) Applicant: The Catholic University Of Korea Industry-Academic Cooperation Foundation, Seoul 06591 (KR)
(72) Inventor: KIM, Tai Gyu, Seoul 06002 (KR); KIM, Su Eon, Yongin-si Gyeonggi-do 16829 (KR)
(74) Representative: Plasseraud IP
(86) International application number: PCT/KR2021/003356
(87) International publication number: WO 2021/187911

(57) **Abstract**

The present invention relates to a cord blood plasma-derived exosome or a mimetic thereof and a pharmaceutical use thereof. More particularly, the present invention provides the use of a human cord blood plasma-derived exosome or an exosome mimetic that mimics the proteomic profile of the cord blood plasma-derived exosome for the improvement, prevention or treatment of various autoimmune diseases or wound healing.

## Description

### [Technical Field]

The present invention relates to a cord blood plasma-derived exosome, a mimetic thereof, and a pharmaceutical use thereof.

### [Background Art]

Human cord blood has been used as a source of hematopoietic stem cells for transplantation, but it contains a number of cells such as immune cells, mesenchymal stromal cells, endothelial precursor cells, and the like. Fetal immune tolerance by the maternal immune system plays an important role in maintaining pregnancy. The immunological interaction between the fetus and the mother represents a two-way communication system that is regulated by the presentation of fetal antigens and/or the recognition of their antigens by a maternal immune system and responses thereto. Human cord blood-derived cells have specific immunomodulatory properties contributing to maintenance of pregnancy. Moreover, human cord blood is also an abundant source of immunosuppressive cells such as regulatory T cells (Tregs) and monocyte-derived suppressor cells (MDSCs).

Exosomes are small cell membrane vesicles that load a unique subset of functional proteins and reflect their cell lineage. Since human cord blood-derived cells are selected and expanded *ex vivo,* their cell-derived exosomes may be effectively isolated from a cell culture supernatant. The exosomes released from the human cord blood-derived cells can induce the differentiation of circulating immunosuppressive cells such as Tregs and MDSCs, and thus may have immunosuppressive effects. However, despite the advance of research on human cord blood-derived cells and exosomes, studies on human cord blood plasma-derived exosomes (CBPexo) are poor. The result of proteomic studies on CBPexo showed that exosome proteins were involved in T-cell proliferation, differentiation and negative regulation; membrane permeability; wound-healing; arginase activity and enzyme regulatory activity. In addition to the human cord blood-derived cells, the human cord blood plasma also includes various immunomodulatory factors, which inhibit IL-2 signaling and inhibit T-cell proliferation. IL-2 and its receptor IL-2R play a role in supporting T cell proliferation and survival and naive T cell differentiation. An IL-2R alpha chain (IL-2Rα/CD25) is an essential component of IL-2R. Moreover, the surface expression of IL-2Rα in CD4+T cells and an IL-2 level in activation T cells are significantly reduced by MMP-9. The inhibition of MMP activity by a chemical inhibitor restores IL-2 production and partially restores T cell proliferation. In addition to MMP-9, CBP immunoregulators are found from CBPexo, and these exosomes exhibit concentrated immunosuppressive activity.

Meanwhile, although skin and soft tissue injuries such as those associated with accidental post-traumatic fractures are common, wound healing requires several steps, such as fibroblast proliferation, collagen synthesis, deposition, angiogenesis, differentiation and migration of immunosuppressive cells. It is a highly planned process involving a well-organized series of cellular and molecular events such as hemostasis, inflammation, proliferation and remodeling. Several treatments have been proposed to promote wound healing, but optimal treatment strategies are still under development. Human cord blood is an attractive source of stem cells for transplantable wound healing. Cord blood donors are pregnant women and there is no risk of a disease since they collect cord blood through standardized procedures. However, the use of stem cells in therapy is limited by certain risks, such as tumorigenesis, thrombosis, and unwanted immune responses. Exosome-based therapies have a relatively low risk compared to stem cell-based therapies. Therefore, although an exosome-based therapy is proposed as a promising wound healing approach, to date, there have been no studies on the protein level of CBPexo for wound healing. Previous studies have been limited to wound healing at the miRNA level.

### [Disclosure]

### [Technical Problem]

The present invention is directed to providing a pharmaceutical use of a human cord blood plasma-derived exosome by identifying the proteomic profile thereof, compared to an adult plasma-derived exosome.

The present invention is also directed to providing an exosome mimetic which mimics the proteomic profile of a human cord blood plasma-derived exosome and a pharmaceutical use thereof.

### [Technical Solution]

To achieve the above purposes, the present invention provides an immunosuppressive or wound healing composition, which includes: cord blood plasma-derived exosomes in which the expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B 12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) is higher than that of adult plasma-derived exosomes as an active ingredient.

The present invention also provides an immunosuppression or wound healing method, which includes administering an effective amount of the cord blood plasma-derived exosome into a subject.

The present invention also provides a medium composition for suppressing the differentiation of Th1 and Th17 cells, which includes: cord blood plasma-derived exosomes in which the expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B 12 (SERPINB 12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) is higher than that of adult plasma-derived exosomes as an active ingredient.

The present invention also provides a method of suppressing the differentiation of Th1 and Th17 cells *in vitro,* which includes: culturing cord blood plasma-derived exosomes with naive CD4+T cells *in vitro,* wherein the cord blood plasma-derived exosomes have higher expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B 12 (SERPINB 12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) than that of adult plasma-derived exosomes.

The present invention also provides a cord blood plasma exosome mimetic derived from a HLA and MIC-null cell line and expressing one or more selected from the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B 12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1).

The present invention also provides an immunosuppressive or wound healing composition, which includes the cord blood plasma exosome mimetic.

The present invention also provides an immunosuppression or wound healing method, which includes administering an effective amount of the cord blood plasma exosome mimetic into a subject.

### [Advantageous Effects]

In the present invention, a human cord blood plasma-derived exosome is used in improvement, prevention or treatment of various autoimmune diseases, and an exosome mimetic mimicking the proteomic profile of the exosome imparts an effective immunosuppressive and wound healing effect.

### [Description of Drawings]

FIG. 1A to 1D show the results of identifying the characteristics of exosome membrane vesicles purified from human cord blood plasma:
FIG. 1A shows the result of flow cytometry analysis of CBPexo bound to latex beads coated with various exosome-specific marker antibodies. For comparison, latex beads coated with a complete exosome preparation are included. Plots represent intensities caused by exosome-specific antibodies (CD9, CD63, CD82 and HSP72) with respect to a corresponding bead-only control.
FIG. 1B shows the result of the dot blot analysis of a CBPexo lysate. Corresponding dots were evaluated using an exosome antibody array kit. Exosome-specific antibody spots provide signals with various intensities. Presented values represent the averages of values obtained from three independent experiments. Error bar = Standard error of the mean (SEM). The "blank" represents a negative control, denoting ϕ, and GM130 represents cell debris. The average intensity is analyzed using ImageJ software.
FIG. 1C shows the result of analyzing the morphological characteristics of CBPexo. It was confirmed that CBPexo has morphological characteristics of being formed in a lipid bilayer structure and a size of 50 to 130 nm using an electron microscope. The scale bar denotes 100 nm.
FIG. 1D shows the result of analyzing the size and particle number of CBPexo. The exosome size is 81 ± 1.4 nm, and the particle number is 1.64×10¹² ± 2.37×10¹⁰ particles/mL.
FIGS. 2A to 2C show the molecular function and biological process associated with a differentially expressed protein:

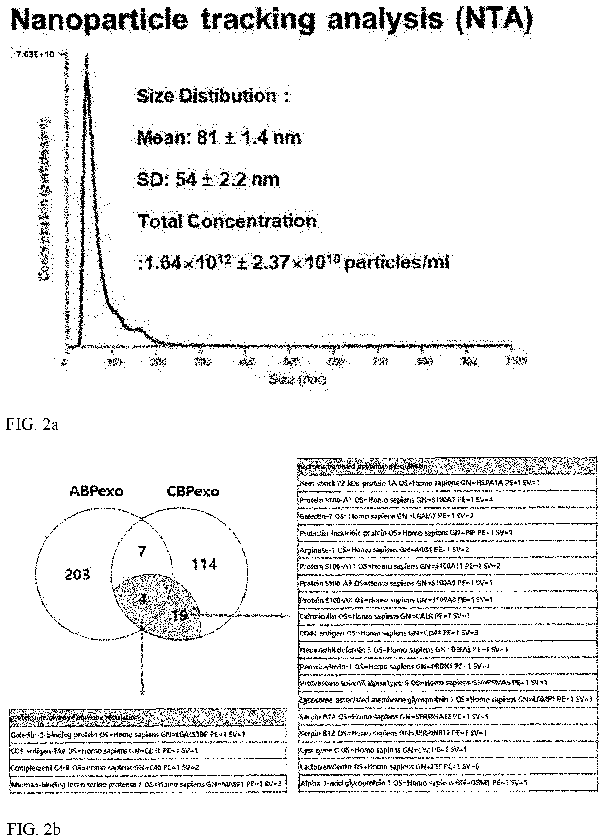

FIG. 2A shows the result of the proteomic analysis for a plasma-derived exosome and various immunosuppressive molecules in screened exosomes through mass spectrometry (testing mass spectrometry data with ExoCarta and Mascot v2.3.01).

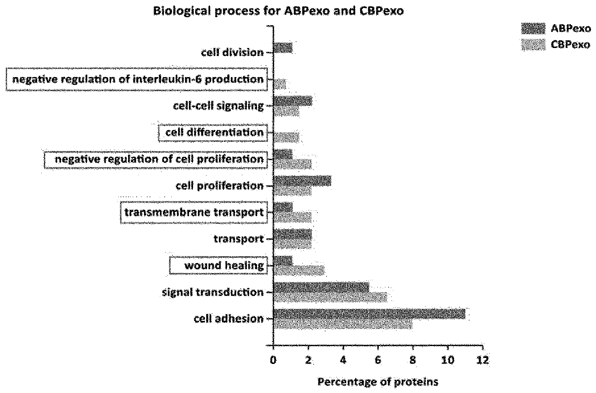

FIG. 2B shows the result of the proteomic analysis for various biological functions of human CBPexo and ABPexo using ExoCarta database and an SBC analysis system.

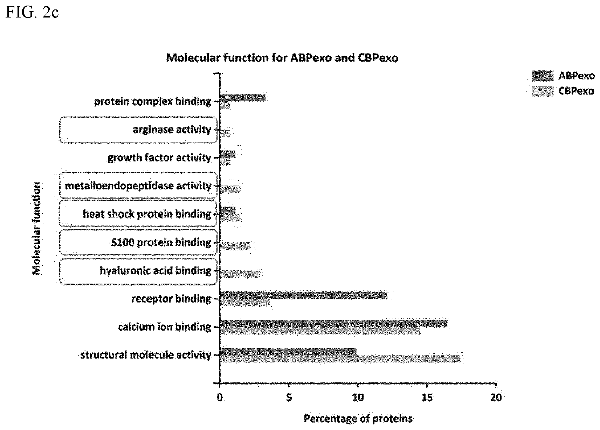

FIG. 2C shows the proteomic analysis result for screened human CBPexo and ABPexo and various molecular functions of immunosuppressive molecules using ExoCarta database and an SBC analysis system. The red box is the functional classification result, indicating a higher association of CBPexo.

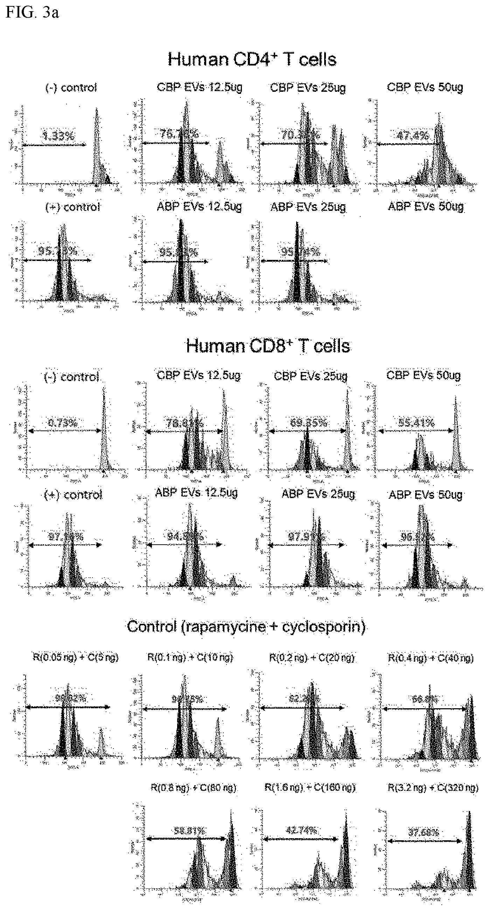

FIGS. 3A to 3D show CBPexo-mediated immunosuppressive mechanisms in CD3/CD28 DYNABEAD-stimulated human CD4+ and CD8+ T cells:
FIG. 3A shows the representative experimental data proving that CBPexo inhibits CD4+ and CD8+ T cell stimulation. The intensity of CFSE-labeled T cells is obtained by flow cytometry, and additionally analyzed using ModFit LT 3.0 software.

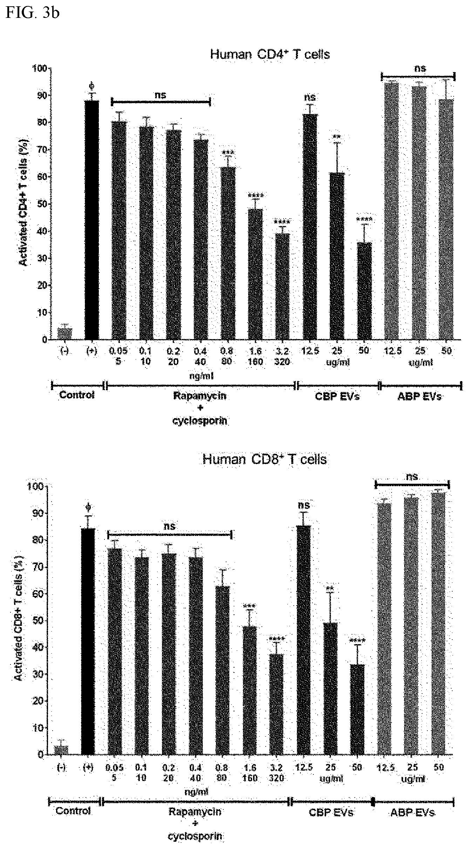

FIG. 3B shows that human PBMCs are activated using CD3/CD28 DYNABEAD in the presence of CBPexo or ABPexo in a concentration-dependent manner. Rapamycin and cyclosporine are well known as immunosuppressants and are used as standard positive controls. The intensity of CFSE-labeled T cells is obtained by flow cytometry, and additionally analyzed using ModFit LT 3.0 software. All experiments are repeated five or more times using five types of different batches of CBPexo (1 batch of plasma exosomes = the sum of 10 units of cord blood). Statistically significant difference (ANOVA test): *p <0.1 **p <0.05 ***p <0.01. Activated PBMCs represent positive control=ϕ.

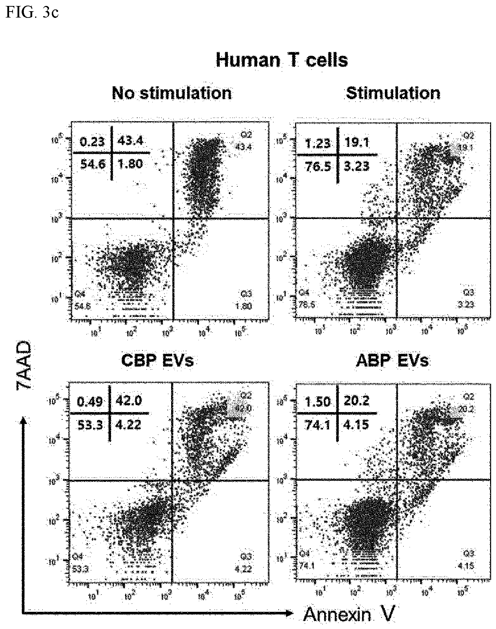

FIG. 3C shows that the representative experimental data of annexin V-FITC and 7AAD show that human PBMCs are activated with CD3/CD28 DYNABEAD in the presence of CBPexo or ABPexo.

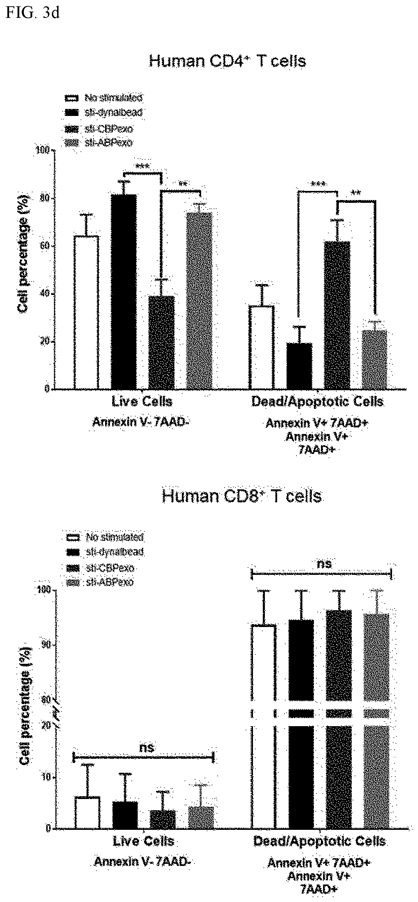

FIG. 3D shows that CBPexo induces the apoptosis of CD4+T cells activated by CD3/CD28 DYNABEAD. CD4+ and CD+8 T cells are collected after 156 hours and stained with annexin V-FITC, 7AAD, anti-CD4-APC and anti-CD8-V450 antibodies. Cells gated on CD4 are illustrated. Analysis data is analyzed using FlowJo v10. This experiment is independently repeated five times. Statistically significant difference (ANOVA test): *p <0.1 **p <0.05 ***p <0.01.

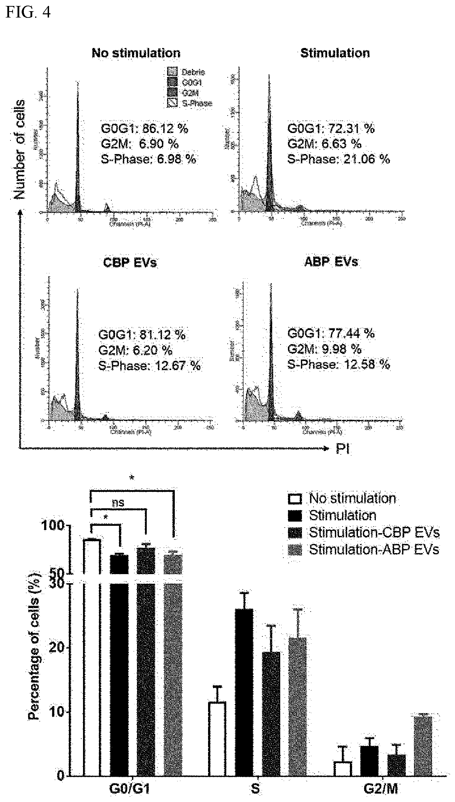

FIG. 4 shows the effect of GO/G1 cell cycle arrest in CD4+ T cells induced by CBPexo. The experiment is repeated independently three times, and significance between groups is calculated using one-way ANOVA: * p <0.1 ** p <0.05 *** p <0.01.

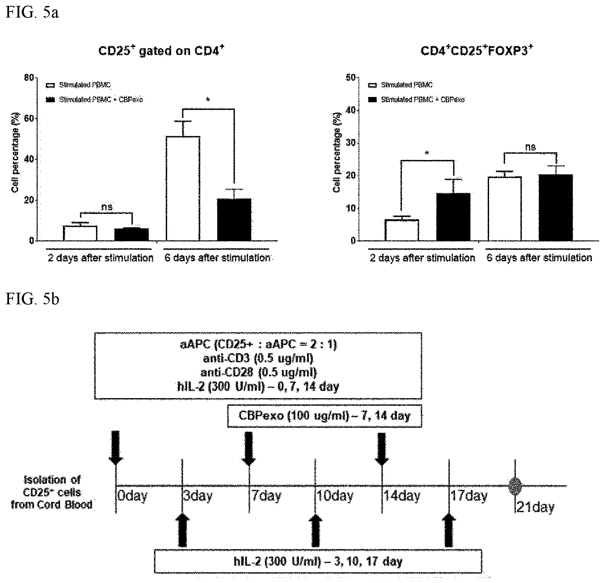

FIGS. 5A to 5E show the effect of CBPexo-induced differentiation of Treg cells and functional upregulation on T cell suppression:
FIG. 5A shows CD4+CD25+ and FOXP3+ expression of PBMCs in the presence of CBPexo. This experiment is independently repeated three times and has statistically significant differences (ANOVA test): *p <0.1 **p <0.05, ***p <0.01.
FIG. 5B shows regulatory T cell expansion or differentiation from cord blood CD25+ cells.

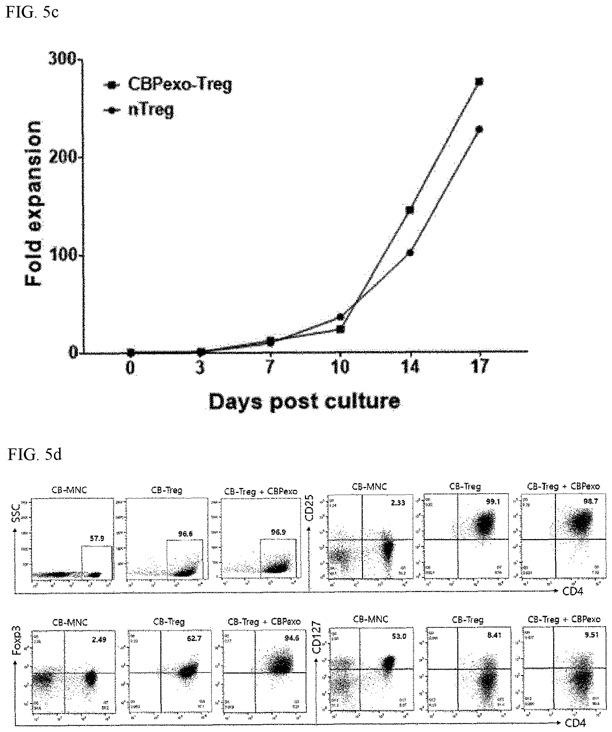

FIG. 5C shows the fold expansion of the same 1 unit of cord blood CD25+ cells by a cytokine, aAPC and an antibody combination in the presence or absence of CBPexo.
FIG. 5D shows the expansion of sorted cord blood CD25+ cells according to anti-CD3/CD28 and IL-2 cocktail and Treg differentiation in the presence or absence of CBPexo. Cord blood mononuclear cells (CB-MNCs) are used as a negative control.

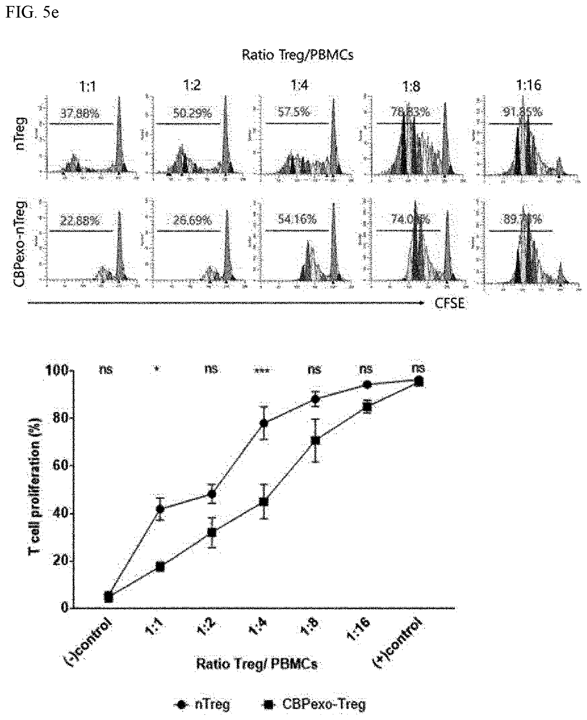

FIG. 5E shows the comparison of CBPexo-induced Treg and nTreg inhibitory functions in PBMCs after DYNABEAD stimulation. The inhibitory functions are evaluated by CFSE dilution. The flow cytometry data obtained from three independent experiments are summarized as a straight line as a ratio of Treg and nTregmediated T cell proliferation induced by CBPexo in PBMCs. The data are expressed as mean ± standard deviation. Statistically significant difference (two-way ANOVA test): *p <0.1 ^{∗∗}p <0.05 ^{∗∗∗}p <0.01(n=4).

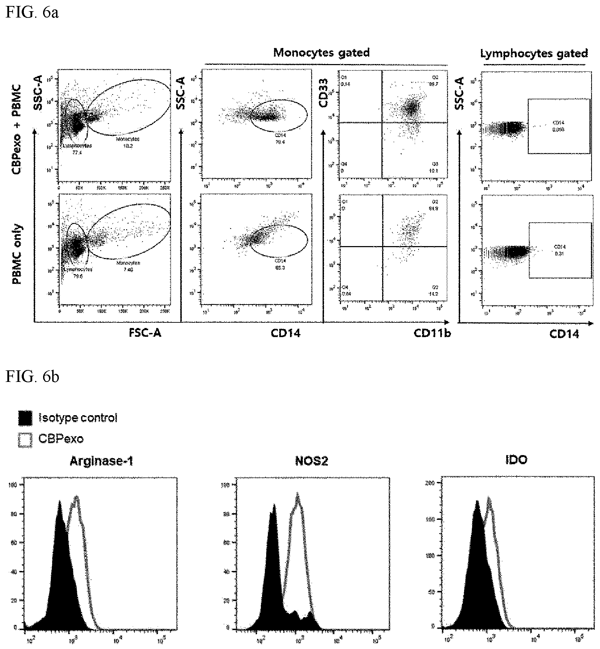

FIGS. 6A and 6B show CBPexo effects caused by the induction of MDSC differentiation:
FIG. 6A shows that non-stimulated PBMCs are cultured in the absence (CTRL) or presence of CBPexo. Gating strategy: To select monocytes (CD14+), physical parameters, that is, forward scattering (FSC) and side scattering (SSC) are used. MDSCs were differentiated by evaluating CD11b/CD33 expression.
FIG. 6B shows that, in the PBMC population, CBPexo promotes the immunosuppressive phenotype of MDSCs and stimulates the production of arginase 1, NOS2 and IDO. The representative FACS histograms for intracellular staining of arginase 1, NOS2 and IDO and the staining of CD14+/CD11b/CD33+ cells are shown.

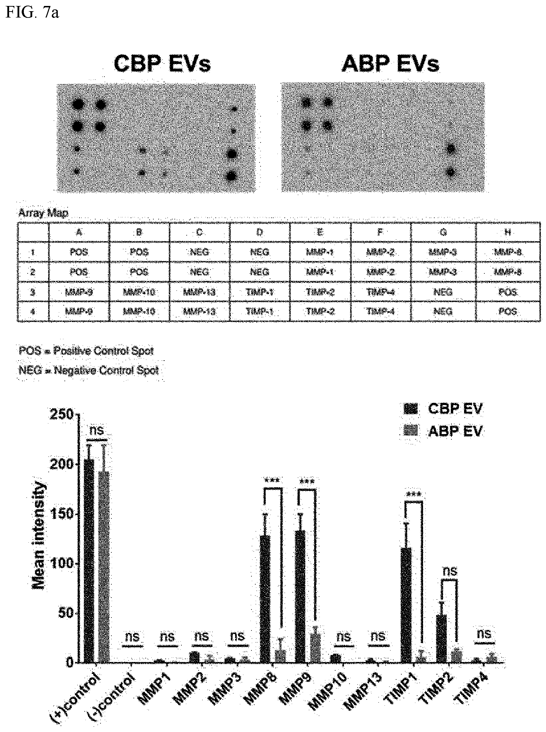

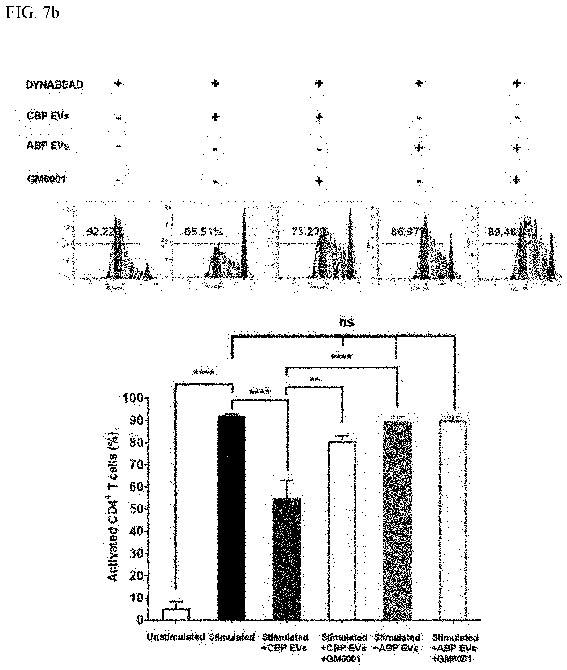

FIGS. 7A and 7B show the effects of CBPexo on the induction of T cell immunosuppression by specific MMP:
FIG. 7A shows the result of analyzing MMP and TIMP antibodies of 300 µg of a CBPexo or ABPexo lysate. Corresponding dots are assessed using an exosome antibody assay kit. MMP and TIMP antibody spots giving various intensities of signals are calculated using ImageJ software. This experiment is independently repeated three times.
FIG. 7B shows that CBPexo-mediated inhibition by MMP inhibition induces partial restoration of T cell proliferation. T cells are treated with GM6001 or not treated before comparison through a CFSE assay at 156 hours after the stimulation by DYNABEAD in the presence of CBPexo. CFSE-labeled cells are obtained by FACSCanto, and gated at a CD4+ event. The ratio of cells in each generation is calculated using ModFit LT4.0 software. This experiment was independently repeated six times. Statistically significant difference (ANOVA test): *p <0.1 **p <0.05 ***p <0.01.

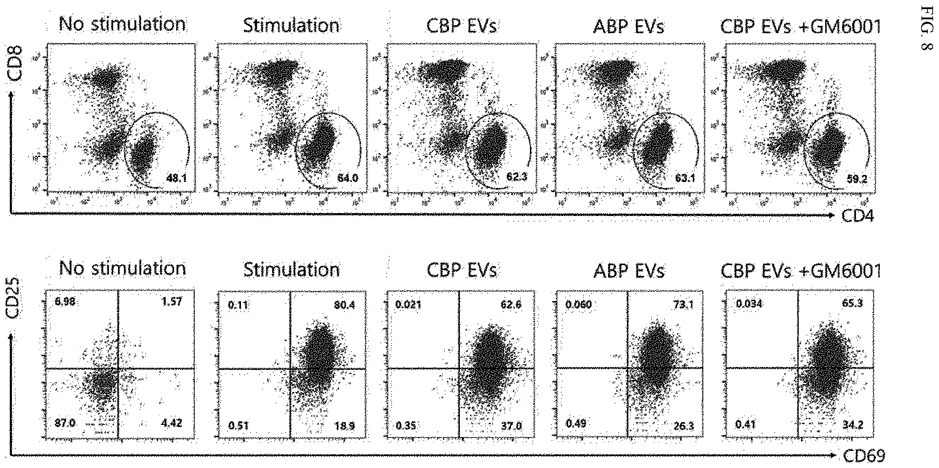

FIG. 8 shows the effects of expressing the activation markers CD25 and CD69 in CD4+T cells by CBPexo treated with GM6001, which is an MMP inhibitor.

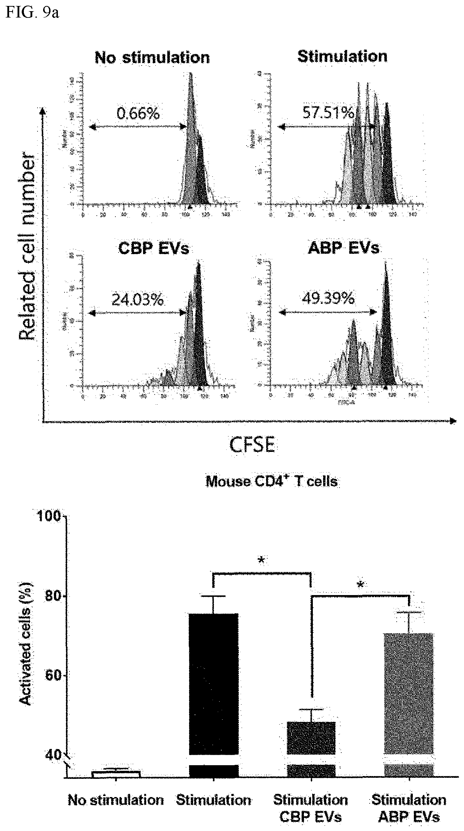

FIGS. 9A to 9E show the mouse cross-reactivity of T cell suppression and IL-2 downregulation by human CBPexo:
FIG.9A shows that the immunosuppressive effect of CBPexo is examined by DYNABEAD-stimulated mouse CD4+T cell proliferation. This experiment is independently repeated five times. Statistically significant difference (ANOVA test): *p <0.1 **p <0.05 ***p <0.01.

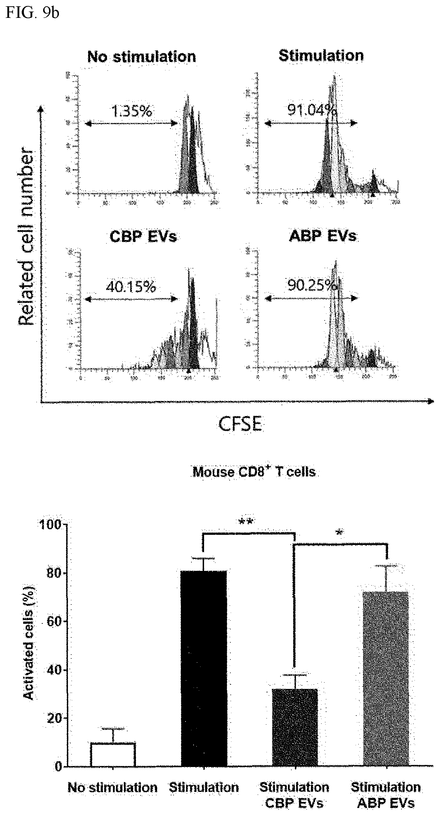

FIG. 9B shows that the immunosuppressive effect of CBPexo is examined by DYNABEAD-stimulated mouse CD8+T cell proliferation. This experiment is independently repeated five times. Statistically significant difference (ANOVA test): *p <0.1 **p <0.05 ***p <0.01.

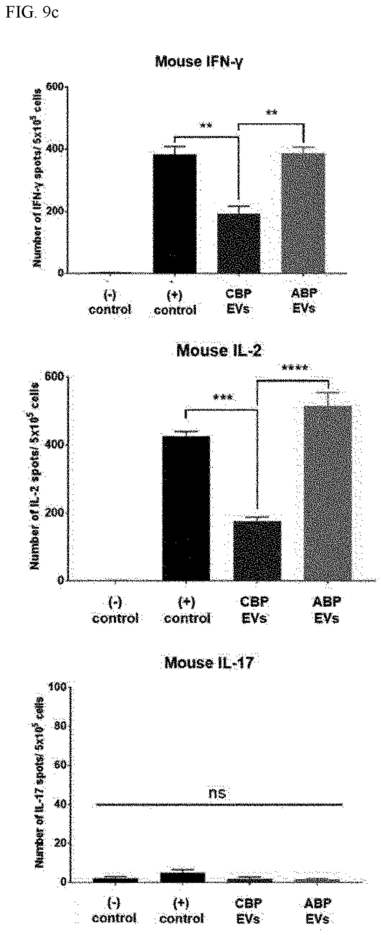

FIG. 9C shows that, to quantify levels of IL-2, IFN-γ and IL-17 secretory T cells, an ELISPOT assay is performed on day 6. Mouse splenocytes are stimulated by CBPexo or ABPexo, and IL-2, IFN-γ and IL-17 secretory T cell patterns are observed.

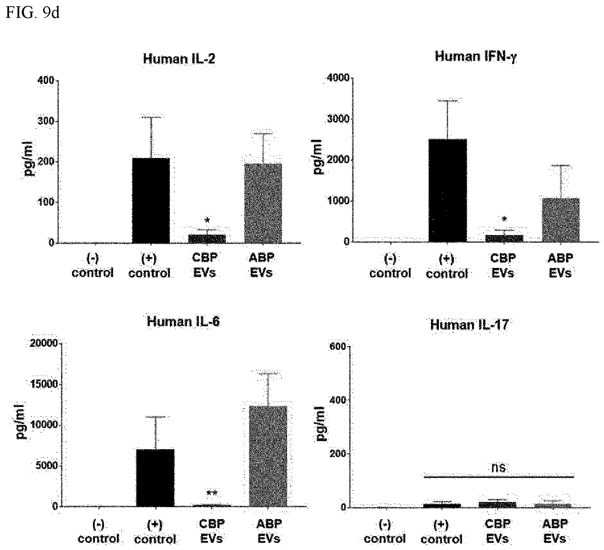

FIG. 9D shows that CBPexo significantly reduces human T cell proliferation by IL-2, IFN-γ and IL-6 production. CBPexo not only cleaves IL-2Rα in human T cells but also downregulates IL-2, IFN-γ and IL-6 secretion associated with the differentiation of Th17 and Th1 cells. The data represents that cytokine production is inhibited by CBPexo, abrogating T cell differentiation capacity. Cytokine levels at 156 hours in the harvested culture supernatant are analyzed using a human cytometric bead array. This experiment is independently repeated three times.

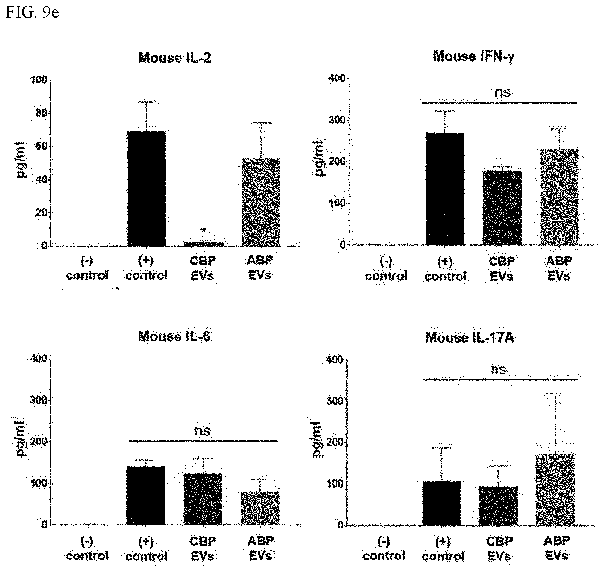

FIG. 9E shows that the changes in cytokine level are observed using a mouse cytometric bead array. As a result, CBPexo downregulates IL-2 secretion in human T cells as well as reduces IL-2 in mice. This experiment is independently repeated three times.

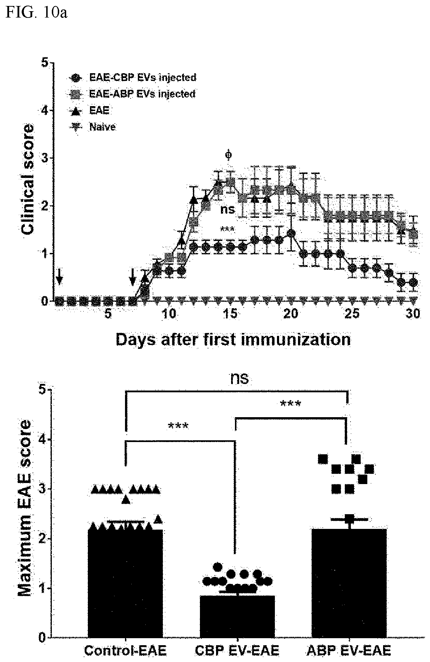

FIGS. 10A to 10C show the changing patterns of Treg and IL-2 levels in exosome-treated experimental autoimmune encephalomyelitis (EAE) mice:
FIG. 10A shows that EAE development is reduced in CBPexo-treated EAE mice. EAE is induced in a total of 15 C57BL/6 mice treated with MOG/CFA and PTx for immunization. CBPexo and ABPexo are injected intravenously twice on day 0 and day 7 into 5 EAE mice at a dose of 100 µg (black arrow). Clinical scores of EAE are observed for 30 days according to the following criteria. Critical score of diseased rats: 0, no sign of disease; 1, limp tail; 2, limp tail and partial weakness of hind limp; 3, complete paralysis of hind limb; 4, complete hind limb and partial front limb paralyses; 5, death. Mice are assigned to different groups to analyze clinical scores after EAE induction: EAE mice (n=5) : CBPexo or ABPexo-inj ected EAE mice (n=5). EAE denotes a positive control=ϕ.

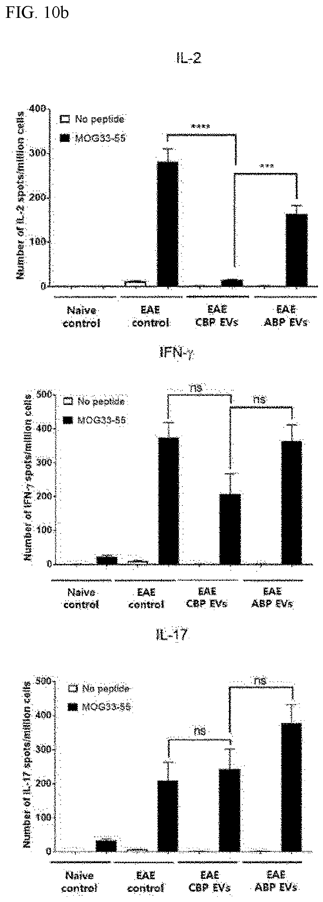

FIG. 10B shows the result of detecting MOG35-55-induced cytokine recall response in whole splenocytes during acute EAE. C57BL/6 mice were immunized with MOG33-55 in CFA and tested 22 days after immunization. All of the tested mice exhibit the clinical symptoms of EAE. The frequencies of MOG peptide-specific IFN-γ, IL-2 and IL-17 secreting cells are measured using an ELISPOT assay to be compared to an EAE control, a CBPexo-treated EAE group and an ABPexo-treated EAE group. MOG peptide-specific T cells are analyzed during re-stimulation with an MOG peptide (25 µg/mL).

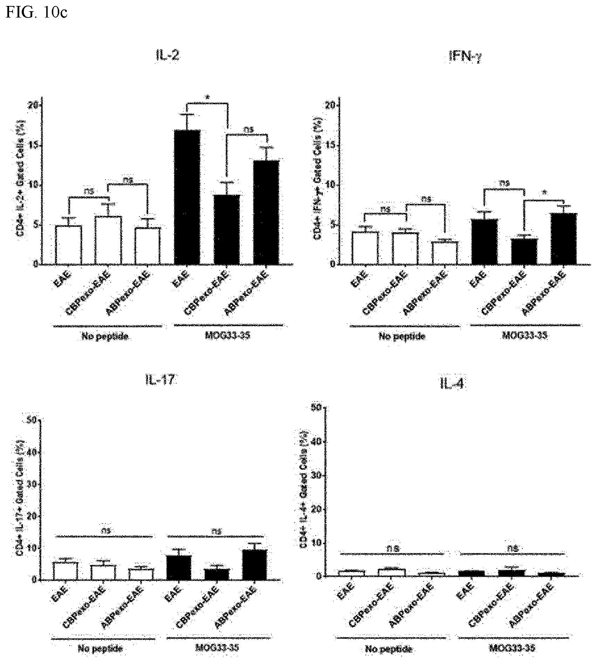

FIG. 10C shows that, in the presence or absence of the MOG33-55 peptide, Th1-, Th2- and Th17-specific cytokine-gated CD4+T cells were measured using cytokine staining in cells and compared with those of the EAE control, CBPexo-injected EAE group and ABPexo-inj ected EAE group (n=4).

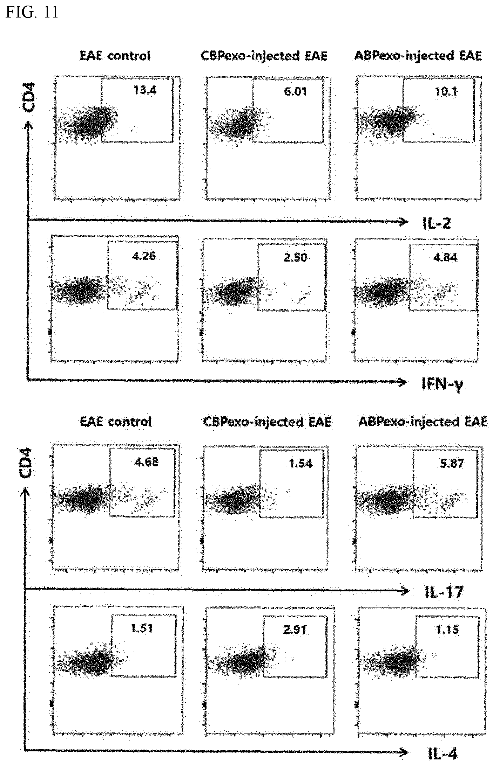

FIG. 11 shows the result of detecting the re-reaction in MOG35-55-induced cytokine in splenocytes of the EAE model.

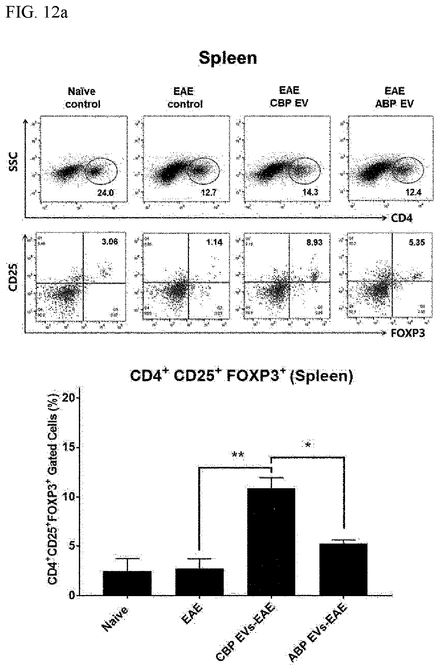

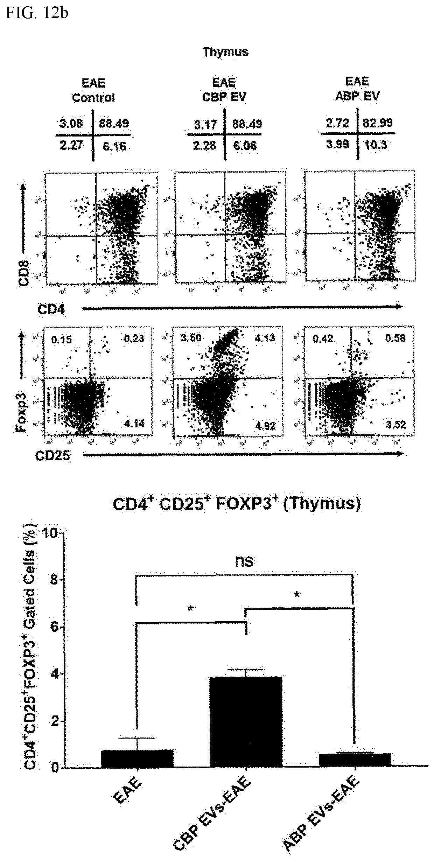

FIGS. 12A and 12B show the patterns of Treg changes in the spleen (FIG. 12A) and thymus (FIG. 12B) of exosome-treated EAE mice. The frequency of CD4+T-gated MOG peptide-specific FOXP3+ cells is compared with those of the EAE control, the CBPexo-treated EAE group and the ABPexo-treated EAE group using a cytokine staining method in cells.

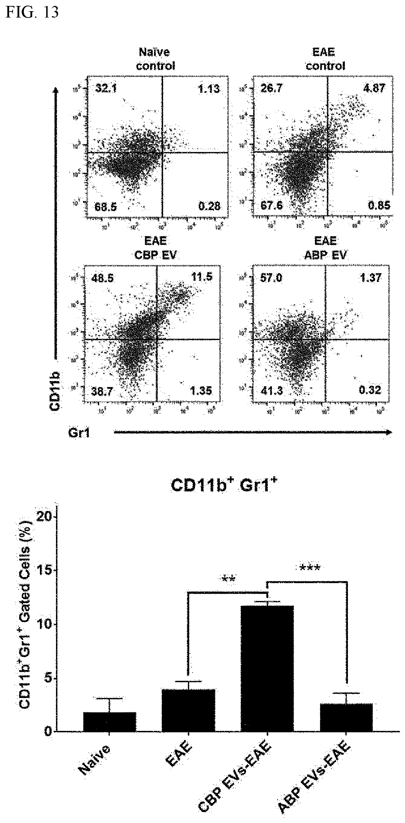

FIG. 13 shows the MDSC population of experimental autoimmune encephalomyelitis (EAE) mice treated with exosomes. The frequencies of CD11b+ Gr1+ cells and CD11b+ NOS2+ gate cells were determined by comparison with the frequency of the MDSC population in an intact control, an EAE control, a CBPexo-injected EAE group and an ABPexo-inj ected EAE group.

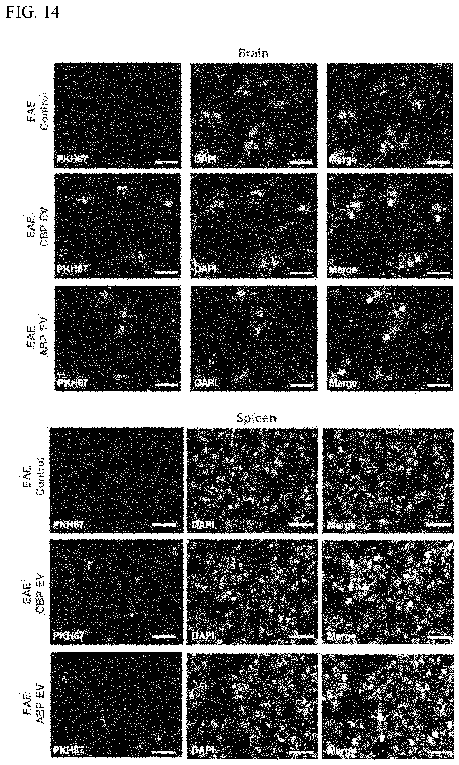

FIG. 14 shows the migration ability of exosomes to the brain and spleen of EAE mice. The EAE control, the CBPexo-treated EAE group and the ABPexo-treated EAE group were compared to see whether PKH-67 fluorescence-stained exosomes are delivered to DAPI-stained tissue.

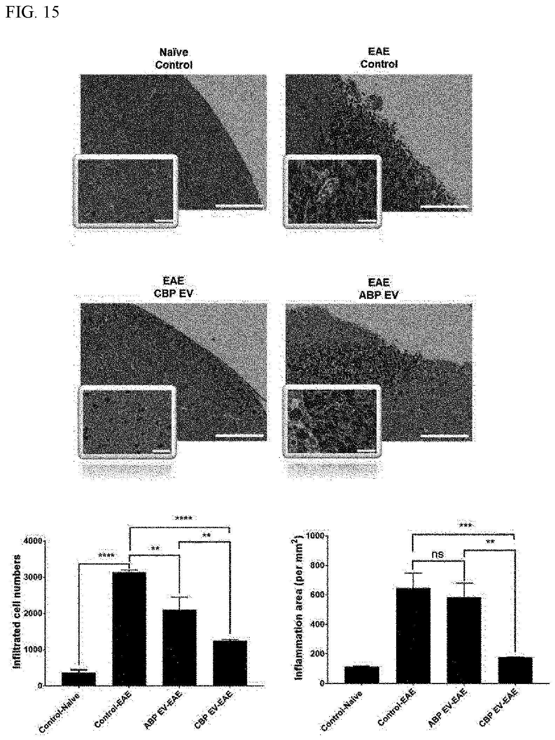

FIG. 15 shows the comparison of the pathologically therapeutic effect by CBPexo in the brain of EAE mice with the EAE control, the CBPexo-treated EAE group and the ABPexo-treated EAE group.

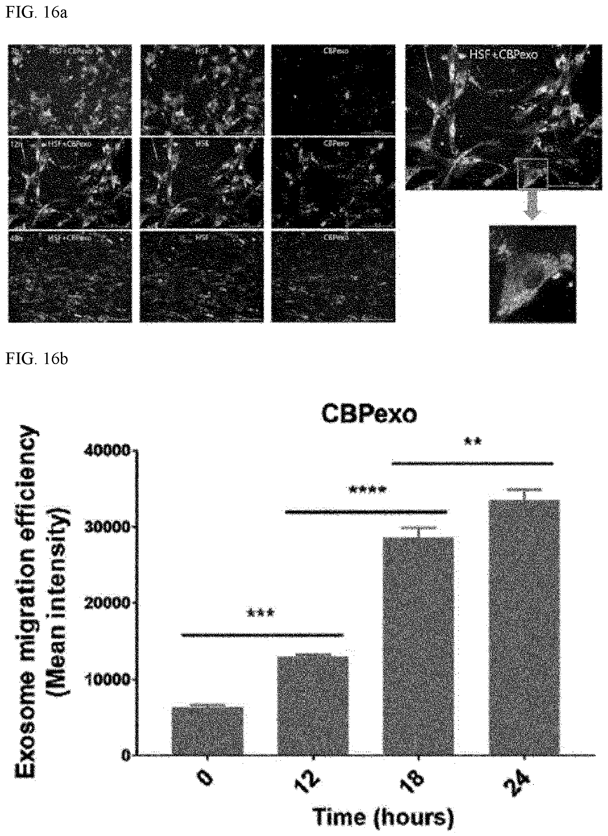

FIGS. 16A and 16B show that CBPexo is internalized by fibroblasts and induces cell proliferation:
FIG. 16A shows that red-labeled CBPexo (100 µg/mL) was significantly expressed in HSF over 3 to 24 hours. The red-labeled exosomes were shown in green-labeled HSF cells.
FIG. 16B shows that red-labeled CBPexo was significantly expressed on HSF over 0 to 24 hours.

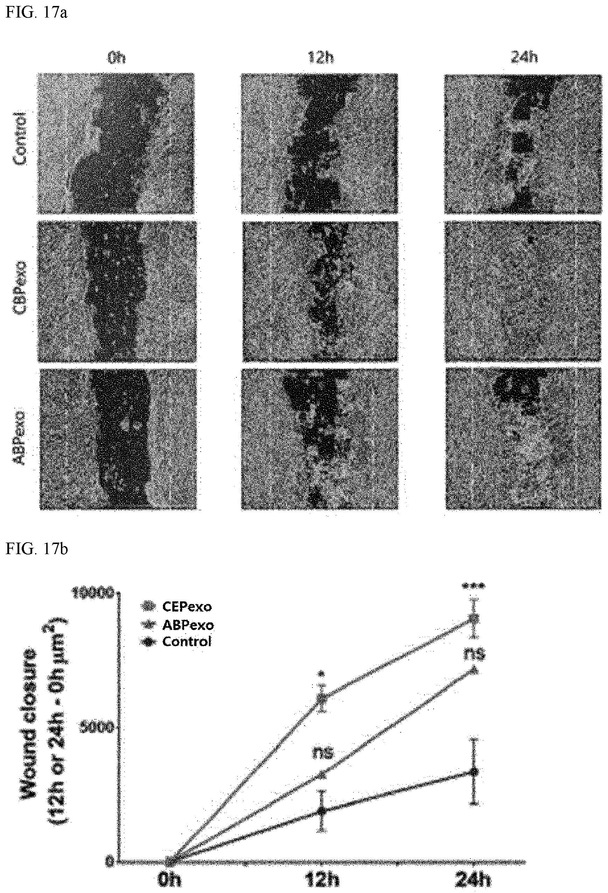

FIGS. 17A to 17D show that CBPexo significantly increases the mobility of HSF compared to ABPexo as shown by scratch wound analysis:
FIG. 17A shows an HSF area in wound closure according to the presence or absence of CBPexo or ABPeox. This measurement was performed over 0 to 24 hours. The fibroblasts in a large area were completely sutured at 24 hours after the addition of CBPex. Here, the concentration of CBPexo and ABPexo was 100 µg/mL.
FIG. 17B shows that the statistical analysis of CBPexo and ABPexo was performed using the scratch wound analysis result. This analysis shows that CBPexo exhibits a higher wound healing effect than ABPexo.

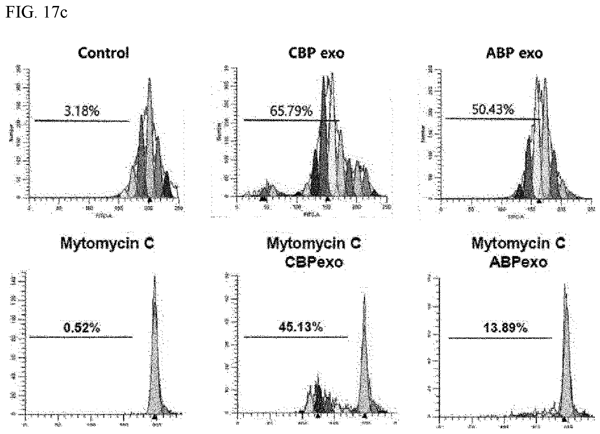

FIG. 17C shows the flow cytometry of CFSE-labeled fibroblasts. The proliferation rate of fibroblasts treated with various combinations of MMC, PBS, ABPexo and CBPexo for 24 hours was measured using ModFitLT 4.1 software.

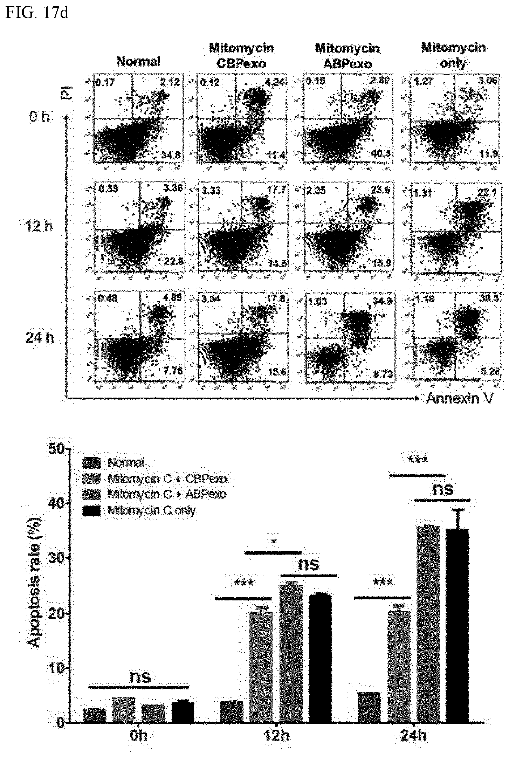

FIG. 17D shows that apoptosis was assessed by double staining of annexin V/propidium iodide. Cells were exposed under various conditions of MMC, PBS, ABPexo and CBPexo for 24 hours. The apoptosis rate was measured by flow cytometry. The statistical analysis of a total of recorded apoptotic cells was performed, and the result is shown as bar graphs.

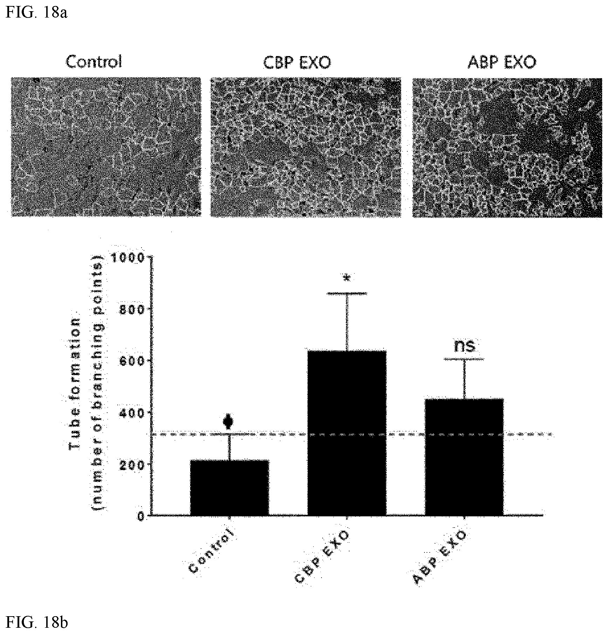

FIGS. 18A to 18B show enhanced endothelial cell tube formation and polarization of M2 Mϕ by CBPexo:
FIG. 18A is a representative image showing *in vitro* HUVEC tube formation in Matrigel obtained after 24 hours using a HUVEC supernatant with or without (control) 20 µg/mL of CBPexo (magnification: 10x). A negative control was obtained from a medium or ABPexo alone.

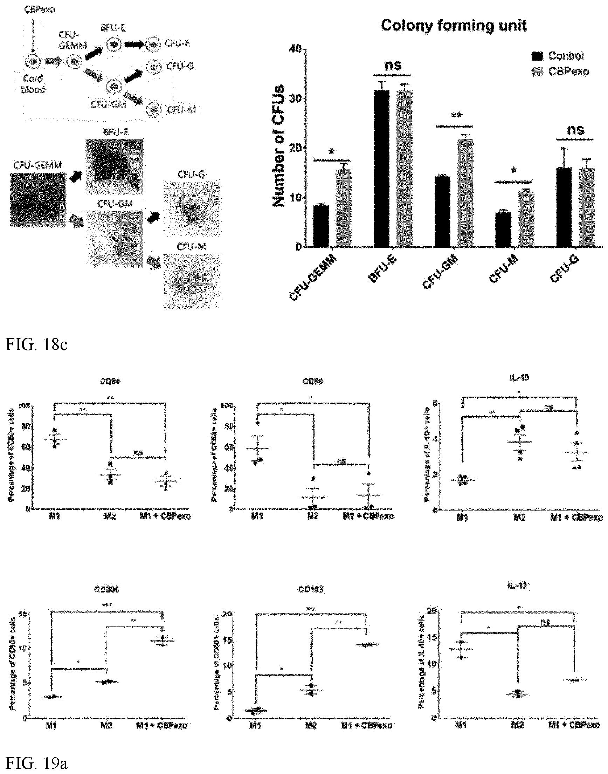

FIG. 18B shows the result of colony-forming unit (CFU) assay using cord blood mononuclear cells 24 and 72 hours after CBPexo treatment. The bars represent CFU number ± SD in two donor samples. These values were obtained from three runs of experiment. Colonies were differentiated into CFU-erythroid (CFU-E), burst-forming unit-erythroid (BFU-E), CFU-GM, CFU granulocyte (CFU-G), CFU-M, and CFU-GEMM using an optical microscope. Statistical significance was established at *p < 0.05, **p < 0.01, ***p < 0.001, and ****p < 0.0001.
FIG. 18C shows the effect of CBPexo on M1 and M2 polarization markers. M1- and M2-polarized macrophages were generated from GM-CSF-treated primary monocytes stimulated with IL-4 depending on the presence or absence of CBPexo. Surface marker and cytokine expression: CD80, CD86, CD206, IL-12 and IL-10 surface levels were compared using flow cytometry. The scatter plots represent the ratio of positive cells from three different donors (dots) with horizontal lines indicating mean values. *p < 0.05; **p < 0.01; ***p < 0.001.

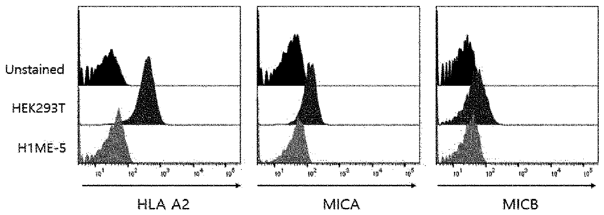

FIGS. 19A to 19H show CBPexo mimetics and the immunosuppressive effects thereof:
FIG. 19A shows the establishment of HLA class I/MIC null HEK 293T(H1ME-5) cell lines using a multiplex CRISPR/Cas9 system. H1ME-5 cells do not exhibit the expression of MICA/B and HLA class I.

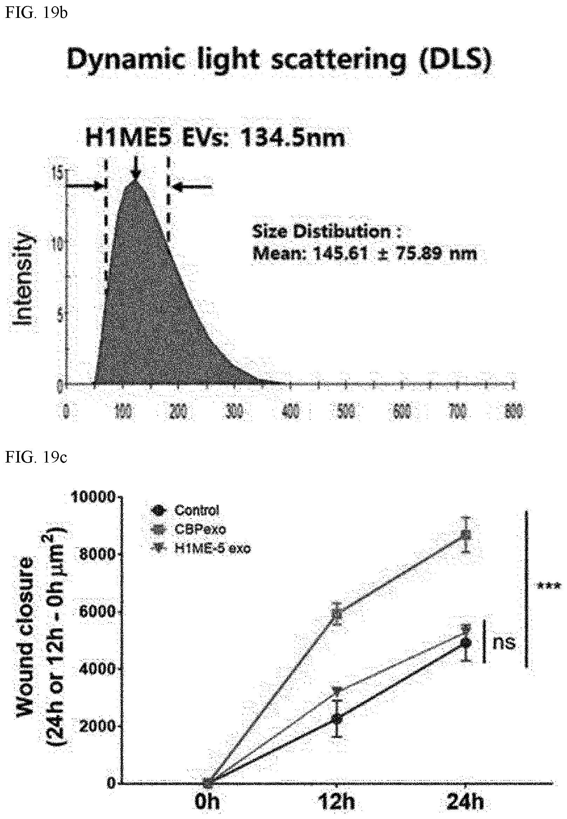

FIG. 19B shows the size distribution of exosomes released from H1ME-5. CBPexo has a size of 145.61 ± 75.89 nm.
FIG. 19C shows that a H1ME-5 cell line was established to generate exosomes with no wound healing effect. The wound-closure area of HSFs was measured in the presence of CBPexo and H1ME-5-exo over 0 to 24 hours. The treatment concentrations of CBPexo and H1ME-5-exo were maintained at 100 µg/mL.

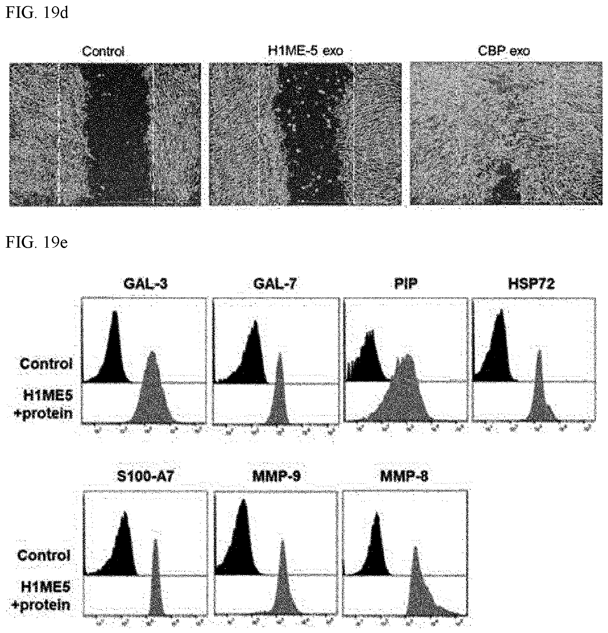

FIG. 19D shows the statistical analysis of the scratch wound assay result.
FIG. 19E shows the expression of GAL-3, MMP-8, MMP-9, PIP, S100A7, GAL-7 and HSP72 included in CBPexo transduced into H1ME-5 cells. Six days after transduction, each molecule-positive cell was sorted using a MoFlo XDP cell sorter.

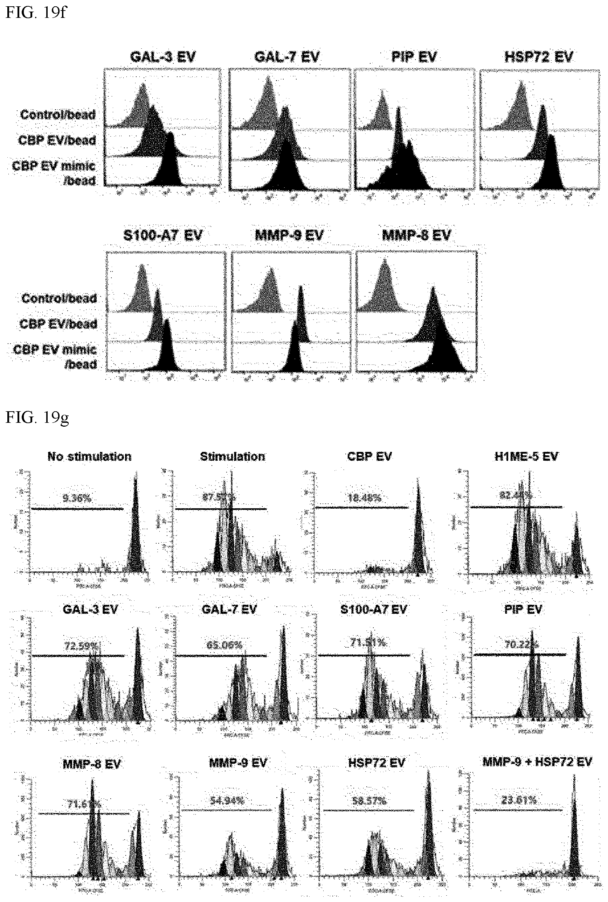

FIG. 19F shows that CBPexo or CBPexo mimetics bound to latex beads coated with GAL-3, MMP-8, MMP-9, PIP, S100A7, GAL-7, and HSP72 antibodies were analyzed by flow cytometry. Intensities caused by GAL-3, MMP-8, MMP-9, PIP, S100A7, GAL-7, and HSP72 antibodies were compared to a corresponding bead-alone control.
FIG. 19G shows representative experimental data proving that CBPexo and an MMP-9-enriched CBPexo mimetic inhibit T cell stimulation. The intensities of CFSE-labeled T cells were obtained by flow cytometry, and additionally analyzed using ModFit LT4.0 software. The concentrations of CBPexo, the CBPexo mimetic and H1ME-5 exosomes are 10 µg/well.

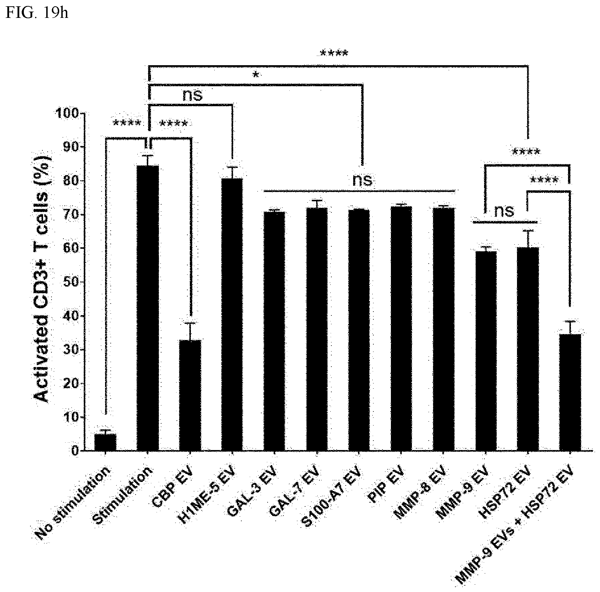

FIG. 19H shows that the statistical values of CBPexo, the CBPexo mimetic and H1ME-5 exosomes are analyzed using a result obtained from CFSE proliferation analysis.

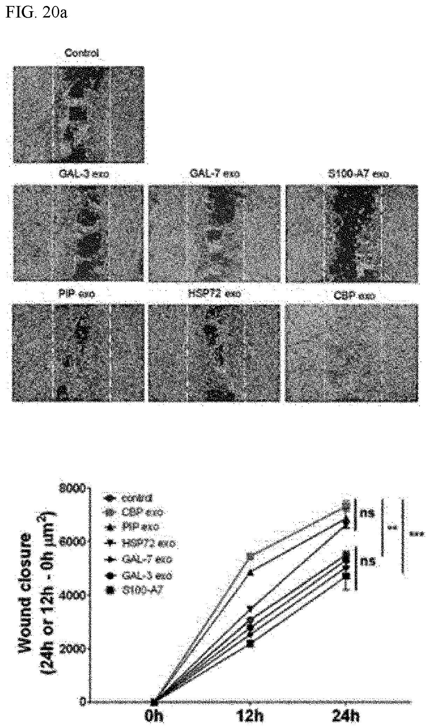

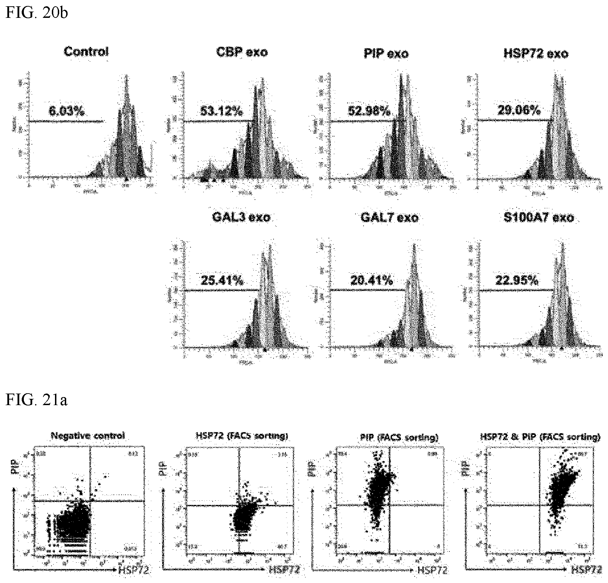

FIGS. 20A and 20B are diagrams showing that artificial exosome promotes wound healing by expressing the selected target molecules:
FIG. 20A shows that the wound healing effect is increased by addition of CBPexo. GAL-3-, GAL-7-, PIP-, HSP72-, and S100-A7-expressed exosomes, playing a critical role in CBPexo, induced the regulation of fibroblasts. HSP72- or PIP-expressed exosomes exhibit a wound healing effect, unlike those in which GAL-3, GAL-7 and S100-A7 are expressed. The scratch wound assay was performed three times, and statistical analysis was performed with GraphPad 7.0 software.
FIG. 20B shows that HSP72, PIP, GAL-3, GAL-7, and S100-A7-expressed exosomes improved fibroblast proliferation. Among these, PIP-exo exhibited the maximum fibroblast proliferation.
FIGS. 21A to 21D are diagrams showing that HSP72 and PIP-expressed artificial exosomesshowed concentration-dependent wound healing and higher wound healing effects when both proteins are M2 Mϕ by HSP72/PIP-exo:
FIG. 21A shows that, six days after transduction, cells simultaneously expressing PIP and HSP72 were sorted using a MoFlo XDP cell sorter and cultured as single cells, thereby obtaining clones with PIP and HSP72.

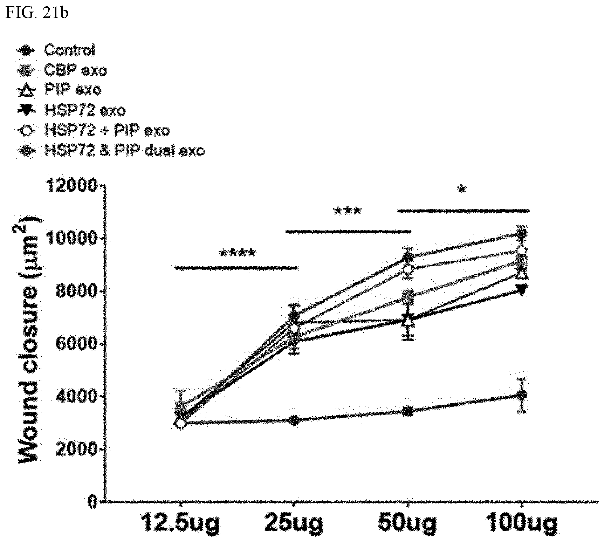

FIG. 21B shows that HSP72- and PIP-expressed exosomes exhibited wound healing effects in a concentration-dependent manner. When used together, HSP72 and PIP-exo exhibited wound healing effects similar to CBPexo. Exosomes simultaneously expressing HSP72 and PIP exhibit wound healing effects similar to that when both HSP72- and PIP-exo are used.

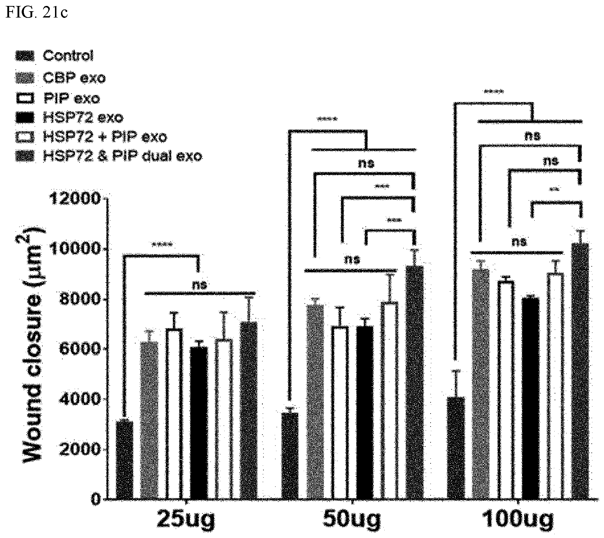

FIG. 21C shows that the combination of exosomes expressing HSP72 and PIP exhibits a wound healing effect similar to CBPexo. The combination of exosomes expressing HSP72 and PIP exhibited a more significant effect than that of when each of HSP72 and PIP was used. Statistical analysis of the CFSE proliferation assay (ANOVA test): *p < 0.1, **p < 0.05, ***p < 0.01, and ****p < 0.001.

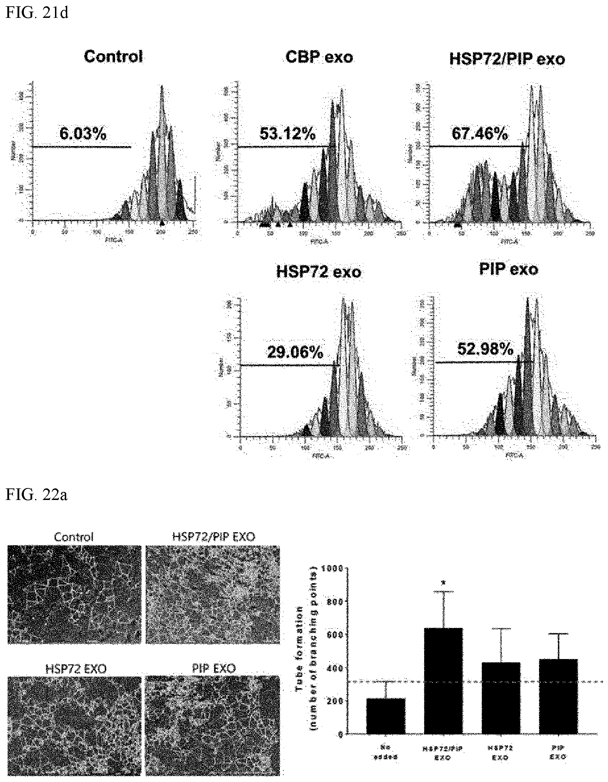

FIG. 21D shows the flow cytometry of CFSE-labeled fibroblasts. The proliferation rates of fibroblasts treated with CBPexo and artificial exosomes for 24 hours were determined using ModFitLT 4.1 software.

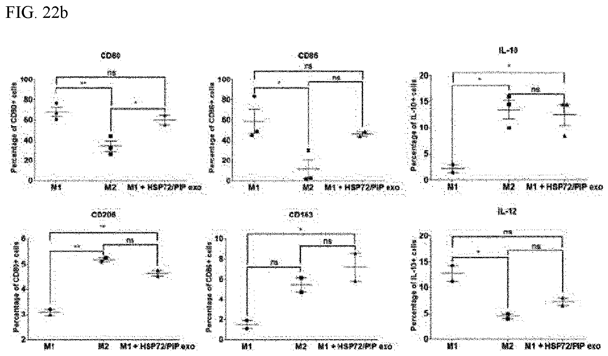

FIGS. 22A and 22B show the enhanced endothelial cell tube formation and the polarization of M2 Mϕ by HSP72/PIP-exo:
FIG. 22A is a representative photograph of *in vitro* HUVEC tube formation on Matrigel obtained after 24 hours using a HUVEC supernatant with or without (control) 20 µg/mL of CBPexo (magnification: 10x). The negative control includes only a medium or ABPexo.
FIG. 22B shows the effect of CBPexo on M1 and M2 polarization markers. M1- and M2-polarized macrophages were generated from GM-CSF-treated primary mononuclear cells stimulated with IL-4 with or without CBPexo. Surface marker and cytokine expression. Flow cytometry analysis for CD80, CD86 and IL-10 expression. Scatter plots represent the ratio of positive cells from three different donor (dots) with horizontal lines indicating mean values for each group. *p < 0.05; **p < 0.01; ***p < 0.001.

### [Modes of the Invention]

Hereinafter, the configuration of the present invention will be described in detail.

The present invention relates to an immunosuppressive or wound healing composition, which includes: cord blood plasma-derived exosomes in which the expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) is higher than that of adult plasma-derived exosomes as an active ingredient.

The present invention also provides an immunosuppression or wound healing method, which includes administering an effective amount of the cord blood plasma-derived exosome into a subject.

The cord blood plasma-derived exosomes (CBPexo) of the present invention include more immunosuppression-related proteins, compared to adult plasma-derived exosomes (ABPexo), and the inhibition of T cell proliferation by factors in CBPexo is caused by apoptosis and cell cycle arrest. In addition, CBPexo treatment increases the CD4+CD25+FOXP3+ Treg cell population and upregulates the function of polyclonal expanded Treg. It is expected that CBPexo arrests a cell cycle by cleaving CD25 that inhibits the downstream of mTOR signaling. Indeed, the secretion of IL-2 and the surface expression of IL-2Rα in CBPexo-treated activation T cells were significantly reduced in CD4+T cells. The blockage of IL-2 signaling by CBPexo plays a pivotal role in the inhibition of the proliferation of CD4+ and CD8+T cells. The blockage of the IL-2 signaling pathway induces the suppression of the immune system and exhibits a therapeutic effect. IL-2/IL-2R interaction activates Ras/Raf/MARP, JAK/STAT and PI3K/AKT signaling pathways in cells to promote the growth, differentiation and survival of T cells.

In addition, MMP-9 of CBPexo is involved in the cleavage of CD25 expressed in T cells when meeting with cancer cells, and MMP-8 also induces disruption of the extracellular domain, resulting in the cleavage of surface molecules associated with immune responses and exhibiting immunomodulatory effects. IL-2Rα cleavage is dependent on MMP-9.

When human CBPexo was applied to a mouse model, an immunosuppressive effect was observed in mouse T cells *in vitro,* confirming the mouse cross-reactivity of human CBPexo.

In addition, the experimental autoimmune encephalomyelitis (EAE) mouse model is comprehensively used to investigate the role of Th cells in disease development. For example, it shows that, when myelin-specific CD4+Th1 cells are transplanted into immature recipient mice, EAE induction is promoted. Similarly, it has been reported that IL-17-secreting T cells, known as Th17 cells, also become a driving force for EAE development. In addition, EAE promotes the initiation of a disease by lowering the frequency of Tregs and impairing their inhibitory functions. The CBPexo of the present invention may change the process of EAE development and regulate the differentiation of antigen-specific CD4+T cells by increasing Treg differentiation and decreasing Th1 and Th17 differentiation *in vivo.*

As described above, the exosomes of the present invention have characteristics of 1) inhibiting T cell proliferation by decomposing IL-2 receptor α (CD25) on the surface of MMP-9-activated T cells expressed in the exosomes and reducing IL-2 production, 2) inducing the differentiation of regulatory T cells (Treg cells) and monocyte-derived suppressive cells (MDSC), and 3) inhibiting the differentiation of Th1 and Th17 cells.

In addition, wound healing requires several steps including fibroblast proliferation, collagen synthesis, deposition, angiogenesis, differentiation, and migration of immunosuppressive cells. The exosomes of the present invention may promote the proliferation of fibroblasts by migrating into human fibroblasts to internalize when the fibroblasts are incubated with the human fibroblasts, which are major effector cells responsible for soft tissue wound healing *in vitro.* These effects are significantly increased compared to adult human plasma-derived exosomes. The effects of the exosomes of the present invention are similarly shown in wound healing. During secondary wound healing, known as a proliferative phase, alternatively activated M2 Mϕ can reduce inflammation and repair wounds by being involved in the proliferation and migration of fibroblasts and tube formation. The exosomes of the present invention also promote macrophage proliferation and induce the differentiation of pro-inflammatory M1 phenotypes into anti-inflammatory M2 phenotypes. When the exosomes of the present invention and activated M1 Mϕ, CD80 and CD86 are incubated *in vitro,* IL-12 expression is downregulated and CD163, CD206, and IL-10 expression is upregulated, it shows that the activated M1 Mϕ is successfully polarized to M2 Mϕ by CBPexo-induced phenotype conversion. Therefore, the exosomes of the present invention may be used as an active ingredient for wound healing.

The term "cord blood plasma-derived exosome" or "CBPexo" used herein are a cell membrane particle derived from cord blood plasma. The exosome has the same meaning as an extracellular vesicle, a circulating microvesicle, or an exosome in the art, and refers to a fragment having a 50 to 100-nm plasma membrane detached from cells. Extracellular vesicles play an important role in mediation of the transport of mRNA, miRNA and a protein between cells and interaction therein. Extracellular vesicles refer to a heterogenous population depending on cells derived, the number of cells, the size of cells and the composition of an antigen. Preferably, in the exosomes of the present invention, the expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B 12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) is higher than that of adult plasma-derived exosomes.

The "active ingredient" used herein refers to a component capable of exhibiting desired activity alone or exhibiting activity in combination with a carrier that is inactive by itself.

In addition, the "immunosuppression" used herein refers to the improvement (decrease in symptoms) and treatment of an immune disease, and the prevention, the inhibition or delay of the disease, caused by a hyperimmune or abnormal immune response. The "immune disease" used herein means a graft-versus-host disease, an autoimmune disease (e.g., rheumatoid arthritis), a hyperproliferative skin disease (e.g., atopic dermatitis, contact dermatitis), a chronic obstructive pulmonary disease (COPD), allergic asthma, bronchitis, allergic rhinitis, or autoimmune hepatitis, but the present invention is not limited thereto.

The wound healing may be improvement or treatment of wound healing, atopic dermatitis, systemic sclerosis, or myocardial infarction. The myocardial infarction is a disease caused by wounds generated in a heart blood vessel, waste products accumulated in the wounded site of the blood vessel, and a blood circulation disorder caused thereby, and the exosomes of the present invention may treat myocardial infarction through wound healing.

In the immunosuppression or wound healing method of the present invention, the subject may be a mammal such as a dog, a cat, a rat, a mouse, or a human, but the present invention is not limited thereto.

The present invention also relates to a medium composition for inhibiting the differentiation of Th1 and Th17 cells, which includes: cord blood plasma-derived exosomes in which the expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B 12 (SERPINB 12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) is higher than that of adult plasma-derived exosomes as an active ingredient.

In addition, the present invention provides a method of inhibiting the differentiation of Th1 and Th17 cells *in vitro,* which includes: culturing cord blood plasma-derived exosomes and naive CD4+T cells *in vitro,* wherein the cord blood plasma-derived exosomes have higher the expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B 12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) than that of adult plasma-derived exosomes.

The "medium" used herein refers to a medium that can inhibit the differentiation of naive CD4+T cells into Th1 and Th17 cells *in vitro,* and includes all of conventional media used in the art, suitable for cell culture. Depending on the type of cells, a medium and culture conditions may be selected. The medium used for culture generally contains a carbon source, a nitrogen source, and trace elements. Examples of such cell culture media include a Dulbecco's Modified Eagle's Medium (DMEM), a Minimal essential Medium (MEM), a Basal Medium Eagle (BME), RPMI1640, F-10, F-12, an α-Minimal essential Medium (αMEM), a Glasgow's Minimal essential Medium (GMEM), and an Iscove's Modified Dulbecco's Medium, but the present invention is not limited thereto.

In addition, the medium may include an antibiotic such as penicillin, streptomycin, or gentamicin.

The medium composition of the present invention may be used alone or in combination with other substances to create a microenvironment for culturing and inhibiting differentiation of Th1 and Th17 cells.

The medium composition of the present invention may additionally add a differentiation inhibitory material as another material, and the differentiation inhibitory material may be any differentiation inhibitory material known in the art, and may be added alone or in combination in various ways according to the status of Th1 and Th17 cells for inhibiting differentiation.

Culturing may be carried out in a CO₂ incubator with 5 to 15% CO₂ aeration rate at 35 to 37 °C, but the present invention is not particularly limited thereto.

The composition of the present invention includes a cord blood plasma-derived exosome in which galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen or arginase-1 (ARG1) is overexpressed, and in order to avoid undue complexity of the specification, overlapping descriptions thereof will be omitted.

The present invention also relates to a cord blood plasma exosome mimetic, which is derived from a HLA and MIC-null cell line and expresses one or more selected from the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1).

The present inventors confirmed that the proteomic profile of CBPexo includes GAL-3, MMP-9, HSP72, PIP, S100A7, GAL-7, LAMP1, SERPINB12, LTF, ORM1, CD5L, C4B, MASP1, PSMA6, PRDX1, DEFA3, CD44 antigen, or ARG1 associated with a pharmaceutical use, for example, immunosuppression or wound healing. However, since it is difficult to isolate sufficient exosomes from cord blood plasma, one or two or more nucleic acids encoding a protein associated with immunosuppression and wound healing are introduced to a H1ME-5 (HLA class I and MIC editing clone-5 cell line) cell line that has neither an immunosuppressive effect nor wound healing ability, and therefrom, exosomes were isolated and purified. According to one embodiment of the present invention, GAL-3, GAL-7, PIP, HSP72, and S100-A7 were selected as such proteins, and exosome mimetics, for example, GAL-3 exo, GAL-7 exo, PIP exo, HSP72 exo, S100-A7 exo, and HSP72 & PIP dual exo were isolated and purified.

Therefore, the term "cord blood plasma exosome mimetic" used herein refers to an exosome mimicking the proteomic profile of the exosome isolated from the cord blood plasma, and particularly, an exosome derived from a cell line having no immunosuppressive effect and wound healing effect, prepared to overexpress a single or plural proteins overexpressed in the exosome for a specific purpose using gene recombination technology. Accordingly, the cord blood plasma exosome mimetic may be prepared by introducing a nucleic acid encoding an exosome protein into a cell line having no immunosuppressive effect and wound healing effect and being isolated therefrom. The nucleic acid is used as the widest meaning, and encompasses singlestranded (ss) DNA, double-stranded (ds) DNA, cDNA, (-)-RNA, (+)-RNA, and dsRNA.

The size of the exosome mimetic may have a diameter of 60 to 250 nm, or 70 to 230 nm.

As the cell line having no immunosuppressive effect, an HLA and MIC-null cell line is used. For example, H1ME-5 (Accession No.: KCTC 13602BP), which an HLA and MICA/B-deficient 293T cell line in which HLAA-A, HLA-B, HLA-C and MICA/B genes are completely removed on the chromosome by deleting exons 2 and 3 of each of HLAA-A, HLA-B, HLA-C and MICA/B using a CRISPR-Cas9 system.

The H1ME-5 cell line was transfected with a vector expressing GAL-3, MMP-9, HSP72, PIP, S100A7, GAL-7, LAMP1, SERPINB12, LTF, ORM1, CD5L, C4B, MASP1, PSMA6, PRDX1, DEFA3, CD44 antigen and ARG1 genes, and a H1ME-5 cell line expressing one or two or more of GAL-3, MMP-9, HSP72, S100A7, GAL-7 and PIP genes are selected and then an exosome (CBPexo mimetic) is isolated.

Accordingly, preferably, the cord blood plasma exosome mimetic of the present invention expresses LAMP1, SERPINB12, LTF, ORM1, CD5L, C4B, MASP1, PSMA6, PRDX1, DEFA3, CD44 antigen, and ARG1.

In addition, the cord blood plasma exosome mimetic of the present invention may express HSP72 and PIP alone or both.

The transfection may be transferred to cells by various methods known in the art, such as microinjection, electroporation, DEAE-dextran treatment, lipofection, nanoparticle-mediated transfection, protein transduction domain-mediated introduction, virus-mediated gene delivery, and PEG-mediated transfection in protozoa, but the present invention is not limited thereto.

The term "vector" used herein means a nucleic acid molecule which is capable of transferring a different nucleic acid connected to a self. As one type of vector, there is a "plasmid," which refers to a circular double-stranded DNA loop, which is capable of ligating an additional DNA segment. As another type of vector, there is a viral vector capable of ligating an additional DNA segment to a viral genome. Some vectors (e.g., a bacterial vector having a bacterial replication origin and an episomal mammalian vector) enable self-replication in host cells when being introduced into the host cells. Other vectors (e.g., non-episomal mammalian vector) may be integrated into the host cell genome when introduced to the host cells and replicated with the host genome. In addition, some vectors may direct the expression of genes that are operably linked to each other. In the specification, such a vector is also referred to as a "recombinant expression vector" (or simply referred to as "expression vector"). Generally, since the expression vector useful for a recombinant DNA IVT mRNA technique generally is a plasmid type, which is the most commonly used vector type, the "plasmid" and the "vector" may be interchangeably used. However, the present invention includes viral vectors providing the same function (e.g., adenovirus vector, adeno-associated virus (AAV) vector, herpes virus vector, retrovirus vector, a lentivirus vector, and a baculovirus vector), and other types of expression vectors such as IVT mRNA. Preferably, a lentivirus vector can be used. Transformation may include any method for introducing a nucleic acid to an organism, cells, tissue or an organ, and appropriate standard technology described above may be selected according to a host cell and carried out as known in the art.

The present invention also relates to an immunosuppressive or wound healing composition, which includes the cord blood plasma exosome mimetic.

The present invention also provides an immunosuppression or wound healing method, which includes administering an effective amount of the cord blood plasma exosome mimetic into a subject.

According to one embodiment of the present invention, among CBPexo mimetics, MMP-9-enriched exosomes are shown as a promising therapeutic molecule for T cell immunosuppression. These results indicate that several multiple effects such as cleavage of CD25 by MMP, the inhibition of IL-2 production, changes in cytokine secretion pattern, and the proliferation of Treg cells are associated with both of T cell differentiation and proliferation. In addition, it is known that exosomes expressing HSP72 inhibit T cell proliferation, and HSP72 upregulates MMP-9.

Accordingly, the cord blood plasma exosome mimetic of the present invention may be used as an active ingredient of the immunosuppressive composition.

In addition, the cord blood plasma exosome mimetic of the present invention significantly improves the migration of fibroblasts, the wound healing effect of an exosome (HSP72 & PIP dual exo) mimetic, which expresses both HSP72 and PIP is higher than PIP exo or HSP72 exo alone in a concentration dependent manner, and better than co-addition of PIP exo and HSP72 exo. In addition, HSP72 & PIP dual exo exhibits better angiogenesis than PIP exo or HSP72 exo alone, and promotes the differentiation of M1 Mϕ into M2 Mϕ, thereby improving wound healing and restoration. Therefore, preferably, the exosome mimetic coexpressing HSP72 and PIP, which mimics the cord blood plasma-derived exosome, may be used as an active ingredient for wound healing.

The immunosuppression is to improve, prevent or treat the above-described immune disease, and overlapping contents associated with an immune disease are omitted in order to avoid excessive complexity of the specification.

The wound healing is to improve or treat the above-described wound healing-related disease, and overlapping contents associated with a wound healing-related disease are omitted in order to avoid excessive complexity of the specification.

In the immunosuppression or wound healing method of the present invention, the subject may be a mammal such as a dog, a cat, a rat, a mouse or a human, but the present invention is not limited thereto.

Meanwhile, the immunosuppressive or wound healing composition of the present invention may include an active ingredient constituting a composition suitable for a diagnostic or therapeutic use *in vitro, in vivo* or *ex vivo,* and an active or inactive pharmaceutically acceptable carrier.

The pharmaceutically acceptable carrier includes any pharmaceutical carriers capable of being mixed with an exosome mimetic such as a protein excipient including a phosphate buffered saline, serum albumin such as human serum albumin (HSA), recombinant human albumin (rHA), gelatin or casein. Examples of carriers, stabilizers and adjuvants are found in Martin REMINGTON'S PHARM. SCI, 18^{th} Ed. (Mack Publ. Co., Easton (1995)) and the "PHYSICIAN'S DESK REFERENCE," 58^{nd}Ed., Medical Economics, Montvale, NJ. (2004). The term "carrier" may include a buffer solution or a pH adjusting agent, and typically, the buffer solution is a salt prepared from an organic acid or a base. Representative buffers include an organic salt such as a salt of citric acid, a salt of ascorbic acid, a salt of gluconic acid, a salt of carbonic acid, a salt of tartaric acid, a salt of succinic acid, a salt of acetic acid, or a salt of phthalic acid; and a tris, tromethamine hydrochloride or phosphate buffer. Additional carriers include a polymeric excipient/additive such as polyvinylpyrrolidone, Ficoll (polymeric sugar), a dextrate (e.g., cyclodextrin, such as 2-hydroxypropyl-, quadrature, -cyclodextrin), polyethylene glycol, an antioxidant, an anti-static agent, a supernatant (e.g., a polysorbate such as "TWEEN 20" or "TWEEN 80"), a lipid (e.g., a phospholipid or a fatty acid), a steroid (e.g., cholesterol), and a chelating agent (e.g., EDTA). An icing inhibitor or freezing point depressing agent may also be included.

The immunosuppressive or wound healing composition of the present invention may be prepared in various suitable formulations. For example, formulations and carriers suitable for parenteral administrations such as intratumoral, intraarterial (in the joint), intravenous, intramuscular, intradermal, intraperitoneal, intranodal and subcutaneous routes include an antioxidant, a buffer, a bacteriostat, a solute that can make a formulation isotonic with the blood of a target recipient, and aqueous and non-aqueous sterile suspending agents that can include a suspending agent, a solubilizing agent, a thickening agent, a stabilizer and a preservative. A preferable method is intravenous or intraperitoneal administration. The dosage of cells administered to a subject is an amount effective to achieve a desired beneficial therapeutic response in the subject over time, or an amount effective in inhibiting cell growth or an amount effective in infection. For example, it may be performed in such a way that a blood sample is obtained from a subject prior to injection and stored to be used in subsequent analysis and comparison. Generally, at least approximately 10⁴ to 10⁶ and typically 1×10⁸ to 1×10¹⁰ cells may be injected intravenously or intraperitoneally into a 70-kg patient over approximately 60 to 120 minutes. For administration, taking into account the overall health conditions and weights of subjects, the exosomes of the present invention are administered at a ratio determined by side effects according to LD-50 (or other toxicity measurement methods) according to a type of cell and types of cells at various concentrations. The administration may be performed at once or at divided doses. The exosomes of the present invention may supplement treatment for other specific symptoms using known conventional therapies including a cytotoxic agent, a nucleotide analogue and a biological response modifier. Similarly, a biological response modifier may be optionally added to treatment with the exosomes of the present invention.

Hereinafter, the present invention will be described in further detail with reference to examples according to the present invention, but the scope of the present invention is not limited by the following examples.

### <Example 1> Preparation and characterization of cord blood plasma-derived exosomes

### 1. Sample preparation

Human peripheral blood monocytes and human cord blood (UCB) were provided by the Catholic Hematopoietic Stem Cell Bank after written informed consent from healthy donors or normal full-term pregnant women. All experiments on human subjects were performed in accordance with the recommendations of the Declaration of Helsinki. The protocol was approved by the Institutional Animal Care and Use Committee (IACUC) of Catholic University. All subjects received written consents to sample donation according to the Declaration of Helsinki.

### 2. Mouse

C57BL/6 mice were purchased from OrientBio, Inc. (Seoul, Korea) and maintained free of specific pathogens in accordance of the guidelines of the Institute of Laboratory Animal Resources of Catholic University, Korea. All animal tests were approved by IACUC of Catholic University, Korea. All animal tests were carried out according to the protocol of the researchers previously approved by IACUC (Approval No. CUMC-2017-0273-05) of the Medical School of Catholic University, Korea.

### 3. Exosome isolation

Human cord blood plasma was sequentially centrifuged at 400× g for 5 minutes and at 2,000× g for 10 minutes to remove cells and cell debris. Subsequently, the cord blood plasma was filtered through a 0.45-µm polyvinylidene fluoride (PVDF) membrane (Nalgene^{™} Rochester, NY). Protein elution was checked by reading the absorbance of each fraction at 280 nm using a NanoDrop spectrophotometer (Thermo Scientific, San Diego, CA). CBP was ultra-centrifuged at 100,000× g for 2 hours, and the cord blood plasma pellet was used for a comparative experiment. As a control, adult blood plasma was isolated and followed the same exosome isolation procedures. All fractions were stored at 4 °C and used within 24 hours for an *in vitro* experiment or frozen at -80 °C. CBPexo was obtained by continuously collecting CBP samples from a total of 10 healthy donors per batch. The characterization of exosomes was performed for each batch using the Exo-Check Exosome Antibody Array (System Biosciences, Palo Alto, CA) or PE-conjugated anti-human CD9 (e-Bioscience, San Diego, CA), anti-human CD63 (BD Biosciences, San Jowe, CA), or anti-human CD82 (Biolegend, San Diego, CA), or anti-human HSP70/HSP72 (Enzo Life Sciences, Farmingdale, NY) FACS antibodies. There was no difference in the characteristics of exosomes between batches.

### 4. Human cord blood Treg expansion

Tregs were purified from 1 UCB unit (The Catholic Hematopoietic Stem Cell Bank) through positive selection using magnetic microbeads to which anti-CD25 was directly conjugated and a manual column (Miltenyi, Bergisch-Gladbach, Germany). CD25+ cells were cultured with a lentivirus-transduced K562 cell line (aAPC) expressing CD80, CD83, 4-1BBL and CD32 in a 2:1 ratio. 10000cGY was applied to aAPC, and the cells were incubated with 0.5 µg/mL of anti-CD3 (OKT3) and anti-CD28 (CD28.2; both are produced by BD Pharmingen, San Diego, CA). Recombinant IL-2 (300 IU/mL; Chiron, Emeryville, CA) was added every three days and maintained during the culture period. The cells were cultured for 14 days and divided every 3 or 4 days.

### 5. LC-MS/MS assay

A half of lysed samples were analyzed through nano LC-MS/MS using the Waters NanoAcquity HPLC system connected to a ThermoFisher Q Exactive HF mass spectrometer. The peptide was loaded onto the trapping column and eluted in a 75 µm analysis column at 350 nL/min using a 2-h reverse-phase gradient: the both columns were packed with a Luna C18 resin (Phenomenex). The mass spectrometer was operated in a data-dependent manner using Orbitrap operated at 60,000 FWHM and 17,500 FWHM for MS and MS/MS, respectively. For MS/MS, 15 types of the richest ions were selected. Mascot DAT files were analyzed with a Scaffold (Proteome Software) to validate, filter and generate unknown lists per sample. Data were filter at 1% protein and peptide FDRs, and at least two specific proteins were required per protein.

### 6. Preparation of CBPexo mimetic

H1ME-5 cells Null-293T (H1ME-5, Accession No: KCTC 13602BP) were cultured in DMEM (Lonza) supplemented with 10% fetus bovine serum (FBS), 1% L-glutamine and 1% penicillin-streptomycin. T2 cells were cultured in RPMI-1640 (Lonza) supplemented with 10% FBS, 1% L-glutamine and 1% penicillin-streptomycin.

The H1ME-5 cells were seeded at 2×10⁶ cells/10 mL in antibiotic-free DMEM (Lonza). After 24 hours, a mixture of 6 types of individual all-in-one plasmids specific for each of 4 targets (GAL-3 (SEQ ID NO: 5), MMP-9 (SEQ ID NO: 1), HSP72 (SEQ ID NO: 4), S100A7 (SEQ ID NO: 6), GAL-7 (SEQ ID NO: 2), and PIP (SEQ ID NO: 3) (NCBI reference sequence; self-produced using pCDH vector)) was transfected into 293T cells using a lipofectamine reagent (Invitrogen, Carlsbad, CA). 48 hours after transfection, the cells were analyzed by flow cytometry. 6 days after transfection, cells positive for GAL-3, MMP-9, HSP72, S100A7, GAL-7 and PIP were sorted using a Moflo XDP Cell Sorter (Beckman), and clones were established.

### 7. Proliferation analysis

Peripheral blood monocytes (PBMC) or splenocytes (5×10⁵) in a culture medium were seeded in an anti-CD3/CD28 DYNABEAD (Invitrogen, Oslo, Norway)-added 96-well tissue culture plate. CBPexo and ABPexo were simultaneously added to appropriate wells. Cell proliferation was measured by 5,6-carboxyfluorescein diacetate-succinimidyl ester (CFSE) staining and flow cytometry. Briefly, 5×10⁵ splenocytes or PBMCs were incubated with 5 µM CFSE (Molecular Probes, Eugene, OR, USA) contained in 1 mL of PBS at 37 °C for 10 minutes, and then washed twice with ice-cold 10% FBS-containing medium. The stained cells were stimulated with DYNABEAD as described above in the presence of CBPexo or ABPexo. After incubation for the indicated time, the cells were obtained using FACSCanto (Becton Dickinson Biosciences, Heidelberg, Germany), and their data were analyzed using Modfit LT 3.0 software (Verity Software House, Topsham, ME). At least three independent experiments were carried out to verify the immunosuppressive effect of CBPexo on activated PBMCs or splenocytes from 10 healthy donors.

### 8. Apoptosis analysis and cell cycle analysis

Apoptotic cells were stained with Annexin V and analyzed according to the manufacturer's instructions. Briefly, 1×10⁶ cells were resuspended in 1 mL of Annexin-binding buffer. APC-Annexin V or FITC-Annexin V (BD Biosciences, San Jose, CA, USA) and 5 µL of working solution of 7AAD (BD Biosciences, Catalog #551076, San Jose, CA, USA) were added to 5×10⁵ cells in 100 µL, and the mixture was cultured at 37 °C in 5% CO₂ for 15 minutes. The analysis data were analyzed using FlowJo v10.

PBMCs were stimulated with DNYNABEAD and cultured in a 96-well flatbottomed plate for 12 to 48 hours. The cells were harvested and fixed in 70% ethanol, and the cell pellet was stained with a propidium iodide (PI; Sigma) solution supplemented with RNase A in PBS. The DNA content was measured with FACS Canto (Becton Dickinson) and analyzed using ModFITLT 4.0 software to measure the sub-G1, G1, S and G2 phases of the cell cycle.

### 9. Protease inhibition

To inhibit protease activity, 10 µg/mL of MMP inhibitor GM6001 (Calbiochem, San Diego, USA) was added to CBPexo and incubated at 21 °C for 2 hours. The GM6001-pretreated CBPexo was used for additional experiments as descried above.

### 10. ELISPOT assay

An ELISPOT assay was performed according to the manufacturer's protocol. Briefly, monoclonal antibodies specific for human IL-2 (Catalog # 551076, BD Biosciences), IFN-γ (Catalog # 551083, BD Biosciences), IL-17 (Catalog # SEL421, R&D Systems) were coated on a microplate (Millipore, Billerica, MA). For an *in vitro* experiment, cells cultured for 6 days were washed three times with a medium, resuspended in 1 mL of a medium and incubated overnight prior to the ELISPOT assay. For an *ex vivo* experiment, MOG33-55 was added to a 96-well microplate at a concentration of 1×10⁶ cells/well in a medium. The microplates were incubated for 20 hours at 37 °C in a CO₂ incubator. Subsequently, the microplates were washed four times with washing buffer. Subsequently, the wells were filled with 100 µL/well of biotinylated polyclonal anti-mouse IL-2, IFN-γ and IL-17. The plates were incubated at 21 °C for 2 hours, washed with buffer, and then incubated at ambient temperature for 1 hour in the presence of 100 µL/well streptavidin-horseradish peroxidase (HRP). Subsequently, an unbound enzyme was removed by washing, and then an enzyme substrate solution was added. After the development of spots, the reaction was stopped by adding distilled water. The plates were inverted and dried overnight away from light. The number of spots corresponding to IL-2-, IFN-γ-, and IL-17-secreting cells was determined using an automatic AID-ELISPOT reader (AID Diagnostika GmbH, Strassberg, Germany).

### 11. Assessment of Th1, Th2 and Th17 cytokines using cytometric bead array (CBA) human and mouse Th1/Th2/Th17 cytokine kits

Human and mouse cytometric bead array (CBA) Th1/Th2/Th17 cytokine kits were purchased from BD Biosciences (Catalog #560484 and #560485). 50 µL of assay beads, 50 µL of a detection reagent and 50 µL of a study sample or the standard material were consecutively added to each sample tube and incubated at 21 °C for 3 hours in the dark. The samples were then washed with 1 mL of washing buffer, and centrifuged at 500 × g for 5 minutes. After discarding the supernatant, the pellet was resuspended in 300 µL of buffer and analyzed on the same day by flow cytometry.

### 12. EAE induction and exosome administration

Encephalomyelitis was induced in mice (n=5 per condition) using a MOG35-55 peptide in a complete Freund's adjuvant (CFA) with pertussis toxin (PTx) as described previously (McCarthy, D.P. et al. Methods Mol Biol, 2012. 900: p.381-401). After the appearance of EAE symptoms, the mice were scored daily by two independent researchers (0, no sign of disease; 1, limp tail; 2, limp tail and partial weakness of hind limp; 3, complete paralysis of hind limb; 4, complete hind limb and partial front limb paralyses; 5, death), stratified and assigned to separate test groups in order to obtain equally weighed average disease scores prior to experimental intervention. Exosomes were injected every week in a "challenged state" at 100 µg/100 µL per mouse.

### 13. Cell culture

Human skin fibroblasts (HSFs, Catholic Hematopoietic Stem Cell Bank) were cultured in high-glucose Dulbecco's modified Eagle medium (Gibco BRL, Grand Island, USA) supplemented with 10% FBS. Cells were maintained at 37 °C with 5% CO₂ in a humidified environment.

### 14. Exosome migration efficiency

To measure CBPexo migration to fibroblasts and endothelial cells, exosomes were labeled with a red fluorescent dye (PKH26; Sigma) and HSFs were stained with a green fluorescent dye (PKH67; Sigma) and incubated at 37 °C for 3 hours.

### 15. Colony-forming unit (CFU) assay

The cord blood team at the Catholic hematopoietic Stem Cell Bank assessed current laboratory practices for CFU when performed on a fresh sample using human cord blood. The experiment was designed to evaluate the differentiation of hematopoietic progenitor cells in UCB. The CFU-C assay was performed using MethoCult^{™} H4435 Enriched medium (STEMCELL Technologies, Vancouver, Canada). Briefly, cells were plated at 1×10⁵ cells/mL into two wells (1 mL/well) in a 24-well plate, and placed in an incubator, which was maintained at 37 °C with 5% CO₂ and a humidity of >85%. A colony was defined as an aggregate of >40 cells. Clusters consisted of 4 to 40 cells. Colony-forming unit-erythroids (CFU-E) were counted after 7 days and burst-forming unit-erythroids (BFU-E) were counted after 14 days using an inverted phase microscope. CFU-granulocyte/macrophage (CFU-G/M) and cluster-G/M were counted after 14 days. The colony number was reported as an average of two wells adjusted to 10⁵ plated mononuclear cells.

### 16. Migration assay

For a scratch wound assay, 5×10⁵ cells/well (three replicates per group) were plated into a 12-well plate and incubated to reach confluence. A monolayer was scratched using a tip and washed with a serum-free medium to remove the detached cells. Subsequently, the cells were cultured in a complete medium supplemented with or without CBPexo (100 µg/well) and GAL-3-, GAL-7-, PIP-, HSP72-, and S 100-A7-expressed exosomes. HSFs were photographed at 0 h, 12 h, and/or 24 h after wounding. The closure area of the wound was calculated as a migration area (µm²). The migration level was observed under Lionheart FX (BioTek Instruments, Inc).

### 17. Treatment of fibroblasts with mitomycin C

A single-cell suspension was prepared by treating fibroblasts with 0.125% trypsin. 5,000 cells in 180 µL medium were seeded into a 96-well plate and incubated for 24 h. In culture for 24 h, the fibroblasts were treated with mitomycin C (MMC) at a concentration of 10 µg/mL. There were four groups of wells: two experimental groups of fibroblasts treated with PBS or MMC only as controls and two experimental groups of fibroblasts treated with MMC, and CBP or ABP exosomes.

### 18. Tube formation assay

An undiluted basement membrane matrix (250 µL; Matrigel, BD Biosciences) was added to each well of a 24-well plate and solidified by incubation at 37 °C for 30 minutes. HUVECs (1×10⁵) were plated onto Matrigel-coated wells each including a different type of exosomes (CBP _{EXO}, HSP72 _{EXO}, or PIP _{EXO}), and incubated at 37 °C with 5% CO₂ for 12 h. After removing the medium, 4% paraformaldehyde (PFA) was added for fixation, and the cells were visualized using an optical microscope, and the formation of tube structures was calculated using ImageJ software.

### 19. Macrophage differentiation

For M1 activation, cells were maintained for 2 days with 40 ng/mL IFN-γ (Peprotech), and for M2 activation, the cells were treated with 20 ng/mL IL-4 (Peprotech) for 2 days.

### 20. Statistical analysis

All experiments were performed with at least three replicates per group, and *in vitro* experiments were repeated at least three times. The data are representative values of these experiments, and expressed as mean ± standard deviation (SEM). The statistical significance of the data was analyzed using Prism ver. 7.0 (GraphPad, San Diego, CA). The means of multiple groups were compared to one-way analysis of variance (ANOVA). Independent-sample-t-tests were used to compare the means of two different groups. Statistical analysis was performed using GraphPad Prism software, and P < 0.05 was considered statistically significant.

### (1) Molecular characteristics of cord blood plasma-derived exosomes (CBPexo)

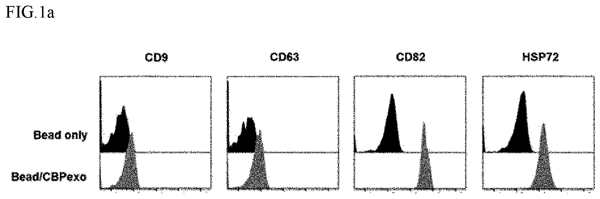

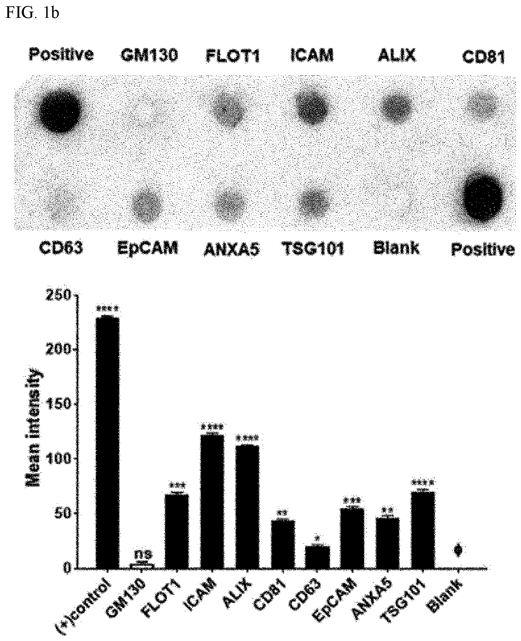

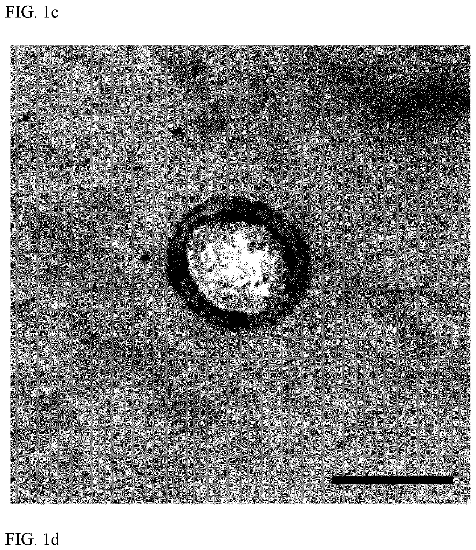

Flow cytometry was used to confirm that CBPexo include typical exosome marker proteins CD9, CD63, CD82 and HSP72. These markers were not included in a bead-only control (FIG. 1A). In addition, the purity of these exosomes was confirmed using an exosome antibody array kit including not only well-characterized exosome protein markers CD81, ICAM, CD63, ALIX, TSG101, EpCAM and FLOT-1 but also a cis-Golgi marker GM130 for cell contamination monitoring. The exosome antibody array kit consists of the standard exosome protein as a positive (+) control and the blank as a negative (-) control. The result obtained from a concentration measurement analysis was graphically represented using the exosome antibody array kit with the ImageJ software, revealing the positive expression of a specific exosome marker. CBPexo were highly positive to FLOT1, ICAM, ALIX, CD81, TSG101, EpCAM and ANXA5, but showed low CD63 expression. As demonstrated by the negative staining for GM130, the isolated exosomes had no cell debris and other contaminants (FIG. 1B).

### (2) Comparison of proteomic profiles of human CBPexo and ABPexo

As a result of liquid chromatography and tandem mass spectrometry (LC-MS/MS), 150 proteins were identified from CBPexo and 214 proteins were identified from ABPexo, and there are at least two mapping peptides per sequence. 11 proteins (4 proteins are immunomodulatory proteins or 7 proteins are not associated with immunomodulatory proteins) of the 150 proteins derived from CBPexo were divided into two subgroups, whereas 19 immunomodulatory proteins were differentially expressed (FIG. 2A). The functional classification of ABPexo and CBPexo was performed using ExoCarta database (Right table of FIG. 2A). The functional classification of ABPexo and CBPexo and additional comparison therebetween emphasized five different biological processes and molecular functions. Some of them are found exclusively in specific pathways such as negative regulation of IL-6, cell differentiation, negative regulation of cell proliferation, transmembrane transport, and wound healing (FIG. 2B). Focusing on the molecular functions of ABPexo and CBPexo, interesting functions related to metalloendopeptidase activity were discovered. It shows that CBPexo are associated with arginase activity, heat shock protein binding, S100 protein binding and hyaluronic acid binding to higher levels than that of ABPexo (FIG. 2C). Therefore, the proteomic analysis of CBPexo reveals that exosomal proteins are associated with immunosuppression and wound healing.

### Table 1 lists the immunomodulator protein in CBPexo.

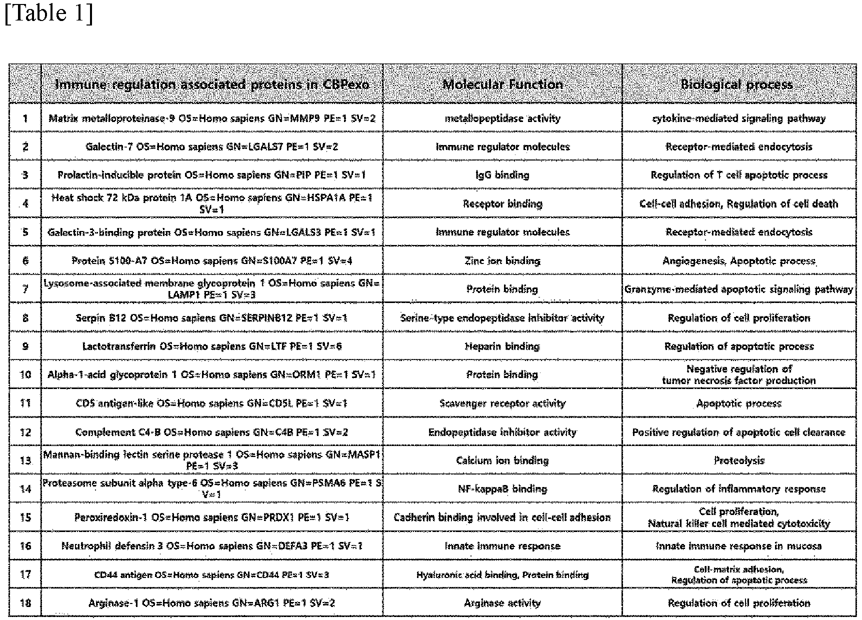

### (3) Inhibitory effect of CBPexo on human T-cell proliferation response

Rapamycin and cyclosporine as positive controls inhibited immunosuppression in a concentration-dependent manner (FIGS. 3A and 3B). In this experimental example, mainly, the immunosuppressive mechanism mediated by the direct contact between CBPexo and immune cells was focused. To solve this, DYNABEAD anti-CD3/CD28 activated PBMCs were cultured with CBPexo or ABPexo to determine whether CBPexo has a potent immunosuppressive effect on activated immune cells. The immunosuppressive effect of CBPexo was investigated by CD4+ and CD8+ T cell proliferation.

As analyzed by CFSE dilution assay, CBPexo did not inhibit CD4+ and CD8+ T cell proliferation, but ABPexo did not (FIGS. 3A and 3B).

To described the fundamental mechanism of the inhibitory effect, apoptotic cell ratio and flow cytometry analysis in the CBPexo or ABPexo groups treated with annexin V and 7AAD were investigated. In addition, it was confirmed whether CBPexo can affect the apoptosis of activated T cells.

As a result, PBMC was activated by DYNABEAD in the presence of exosomes. The apoptosis of T cells was measured by flow cytometry at 156 hours. Annexin V and 7AAD double negative staining indicates live cells, and 7AAD and annexin V positive staining indicates dead cells and apoptotic cells, respectively. As a result, in the CBPexo-treated group, the apoptosis rate was increased compared to the ABPexo-treated group. However, compared to the positive controls, the exosome-treated groups did not show any difference in initial apoptosis period. Moreover, compared to the apoptosis of CD8+ T cells, there was no difference between the CBPexo-treated group and the control. These results show that the apoptosis of CD4+ T cells is increased in CBPexo-treated PBMCs, but there is not significant effect on CD8+ T cells (FIGS. 3C and 3D).

From the above results, it was assumed that the decrease in proliferation response could be caused by the induction of cell cycle arrest. Therefore, cell cycle analysis was performed by measuring the DNA content of each cell group by flow cytometry after PI staining. PBMCs were stimulated with DYNABEAD in the presence of CBPexo or ABPexo, incubated for 156 hours and then stained with PI.

While the cell ratio of the G0/G1 phase showing apoptosis was higher in the CBPexo-treated group than the control, the different was not significant. Moreover, the frequency of cells in the sub-G2/M and S phases was higher in the CBPexo-treated group than the control at 156 hours (FIG. 4). These results suggest that CBPexo reduces T cell proliferation by inducing the G0/G1 cell cycle arrest as well as apoptosis.

### (4) Effect of inducing immunosuppressive cell differentiation in vitro by CBPexo

From the above result, it was assumed that the inhibitory effect of CBPexo observed in T cells in entire PBMC population is caused by the induction of immunosuppressive cells. As the experimental result, the incubation with CBPexo increased the ratio of CD4+/CD25+/FoxP3+ cells in PBMCs stimulated on day 2 (FIG. 5A). However, CD25+ cells gated based on the proportion of CD4+ T cells were significantly downregulated in the presence of CBPexo on day 6, indicating that the IL-2 receptor alpha chain and a factor downregulating CD25 are present in CBPexo; and the present inventors have shown through the previous reference that MMP9 or MMP2 plays the aforementioned role.

To further test the CBPexo effect associated with regulatory T cells, CBPexo was co-cultured with cord blood-derived CD25+ cells, and the cells were incubated for 21 days before the detection of CD4+/CD25+/FoxP3+ and CD127^{low} cells. The cells were effectively expanded by the combination of artificial antigen-presenting cells and anti-CD3/CD28 and IL-2 (FIG. 5B). Treg and CBPexo-Treg cells were generated from the same 1 unit of cord blood, and 17 days after culture, the 16.32-fold increase was shown in the CBPexo-Treg (FIG. 5C). Foxp3 expression increased the cord blood CD25+ cells expanded in CBPexo-induced Treg, compared to the expression in nTregs. However, there was no difference in the expression of CD25 and CD 127 on day 21 after CBPexo and Treg culture. Therefore, CBPexo induces Foxp3 upregulation on day 21 after Treg differentiation (FIG. 5D). To investigate whether CBPexo-mediated signaling enhances the inhibitory characteristics of nTregs, the difference in immunosuppression between nTregs and CBPexo-induced Tregs was tested using CFSE staining. Moreover, CBPexo-derived Tregs were potent in almost all tested Treg:PBMC ratios. The inhibition of >50% was observed at a Tregs:PBMC ratio of 1:4, demonstrating the strong inhibitory ability of CBPexo-derived Tregs (FIG. 5E). Therefore, CBPexo can directly inhibit the immunity of T cells, but it can also induce the differentiation of Tregs at the early stage and participate in improving the functionality of cord blood Treg cells.

For characterization of the phenotypes of inhibitory monocytes in the PBMC population, CD33, CD11b and CD14 were assessed by staining the cells treated with CBPexo for 24 hours (FIG. 6A). As a result, CBPexo stimulated arginase-1, NOS2 and IDO expression in CD33+CD11b+CD14+ cells, which are MDSC population (FIG. 6B). These results reveal that CBPexo not only directly participate in immunosuppression but also induce immunosuppressive cells.

### (5) Restoration of T cell proliferation by inhibition of MMP expressed in CBPexo

Factors in CBPexo that can inhibit T cell proliferation were identified. CD25 was cleaved through proteolysis by MMP-9 and showed the immunosuppressive effect on T cells. Such zinc-dependent endopeptidases are essential factors for cell invasion through the extracellular matrix. To measure the presence of MMP in CBPexo, various MMPs and TIMPs were assessed in CBPexo using human MMP antibody assay.

As shown in FIG. 7A, despite the expression of MMP inhibitory molecules, such as TIMP1 and TIMP2, CBPexo significantly contained high concentrations of MMP9 and MMP8. Therefore, it was assumed that CBPexo cleaved membranebinding IL-2Rα (CD25) by MMP9 and inhibited the proliferation of activated T lymphocytes. To further confirm whether MMP is involved in immunosuppression, prior to the incubation with human T cells, CBPexo was incubated with GM6001, which is an inhibitor for various MMPs including MMP-1, -2, -3, -8 and -9, for 2 hours. Human CD4+T cells cultured with the GM6001-treated CBPexo lead to the restoration of cell proliferation (FIG. 7B).

In addition, CD4+ and CD8+T cells cultured with the MMP inhibitor GM6001 (10 µg/mL)-treated CBPexo showed the restoration of CD25 expression, but there was no difference in activation marker CD69 (FIG. 8). These results reveal that MMP-9 is mainly associated with the immunosuppression of CBPexo induced by the inhibition of CD25 by MMP-9.

### (6) Confirmation of immunosuppressive function of CBPexo in mouse system

CBPexo showed a potent immunosuppressive effect on mouse splenocytes and human PBMCs. The immunosuppressive effect of CBPexo was tested by the proliferation of DYNABEAD-stimulated mouse CD4+ and CD8+T cells. As analyzed by the CFSE dilution assay, CBPexo inhibited CD4+ and CD8+T cell proliferation, but ABPexo did not (FIGS. 9A and 9B). Human CBPexo exhibited mouse cross-reactivity of the inhibition of proliferative T cells. Therefore, these results reveal that CBPexo can be applied to EAE, which is an autoimmune disease animal model.

### (7) Effects of CBPexo in regulating IL-2 signaling and Th1 and Th2 cellassociated cytokines

To expand and response T cells to CBPexo, the cells were cultured (at 1×10⁵ cells/well in a 96-well culture plate) with anti-CD3/CD28 for 6 days. The cells in each well were washed and rested, and then used for ELISPOT assay.

As a result, when co-cultured with CBPexo, the population of IL-2 and IFN-γ-secreting cells was significantly reduced, but IL-17-secreting cells had no difference among the control, CBPexo-treated group and ABPexo-treated group (FIG. 9C). CBPexo inhibited IL-2 and IPN-γ-secreting cells and exhibited an immunosuppressive function.

To assess cytokine secretion, IL-2, IFN-γ and IL-6 levels were assessed using the cytometric bead array. As a result, CBPexo downregulated human IL-2, IFN-γ and IL-6 associated with Th1 and Th17 cell differentiation and the occurrence of autoimmune diseases (FIG. 9D). However, as a result of the assessment of the culture supernatant, when cultured with CBPexo in mouse T cells, only the concentration of IL-2 was significantly reduced (FIG. 9E). These data indicate that the inhibition of IL-2 production by CBPexo is the critical mechanism of T cell inhibition in both human and mouse.

### (8) CBPexo effect to induce reciprocal regulation of Th1 and Th17 cell differentiation to regulate IL-2 cytokine level and alleviate EAE symptoms

As shown in FIG. 10A, the clinical score of CBPexo-treated EAE group was significantly lower than those of the ABPexo-treated EAE group and the EAE control. These results reveal that CBPexo alleviate autoimmune symptoms. To confirm whether CBPexo have immunoregulatory functions in helper T cells, by the ELISPOT assay, cytokine expression levels in splenocytes were measured. The frequency of MOG peptide-specific IFN-γ, IL-2 and IL-17-secreting cells was determined for comparison among the EAE control, CBPexo-treated EAE and ABPexo-treated EAE groups by the ELISPOT assay.

As a result, the MOG peptide-specific IL-2 cells were significantly reduced in CBPeox-treated EAE group, but there was no difference in IFN-γ and IL-17-secreting cells, which was similar to the *in vitro* analysis result (FIG. 10B).

Donor CD4+T cells play a pivotal role in regulating the T cell immune response and reciprocally differentiate into Th1, Th2 and Th17 cells that mediate EAE and the balance of Th subsets. Therefore, their ability to reduce Th1, Th2 and Th17 cellassociated cytokines by CBPexo was further investigated. To confirm this, the cytokine levels were assessed using the Th1, Th2 and Th17 cytometric bead array. As a result of assessing the concentration of the secreted proteins, CBPexo administration induced decreases in IFN-γ, IL-2, IL-6, IL-17 and IL-4 levels compared for the EAE group (FIG. 10C).

To compare the cytokine profiles of Th1, Th2, Th17 and IL-2 in the EAE model, intracellular staining was performed. As a result, the proportion of IL-2 was 2-fold reduced, and IFN-γ and IL-17 were reduced 1 to 4% in the CBPexo-treated group compared to the ABPexo-treated group (FIG. 11). These results demonstrate that CBPexo administration regulates IL-2 signaling, induces reciprocal regulation of the differentiation of Th1 and Th17 cells and thus has a protective effect on EAE.

### (9) CBPexo effect on induction of immunomodulatory cell differentiation in EAE model

The frequency of CD4+CD25+FOXP3+ Treg cells was measured and compared among a non-treated control (negative control), the EAE control (positive control), the CBPexo-treated EAE group and the ABPexo-treated EAE group.

As a result, the Treg population increased in CBPexo-treated EAE group compared to the controls (FIGS. 12A and 12B). These results demonstrate that CBPexo exhibit an immunosuppressive effect by not only inhibiting IL-2 signaling, but also increasing the CD4+CD25+FOXP3+ Treg population *in vivo.* The phenotypes of CD11b+Gr-1+ cells (mouse MDSCs) were expanded during EAE disease progression in CBPexo-injected mice (FIG. 13). MDSCs enriched and accumulated in splenocytes of CBPexo-injected EAE mice play a regulatory role.

### (10) Effects of CBPexo locally and systemically acting in EAE model

To confirm the effect of CBPexo locally and systemically acting, PKH67-labeled CBPexo or ABPexo was injected into the tail vein of an EAE mouse. EAE models may locally or systemically co-occur in the brain. Therefore, cell nuclei of tissues of the brain, which is the most important organ, the spleen, which is the most important lymph organ, in the occurrence of a disease were stained with DAPI, and PKH67-labeled CBPexo was detected in both the spleen and brain tissues (FIG. 14).

Through histopathological examination, as a result of comparing the number of cellular infiltrations and inflammatory cell area in the brains of CBPexo- or ABPexo-treated EAE mice and EAE mice, and normal mice, the cellular infiltration and inflammatory cell area were reduced in the CBPexo-administered group (FIG. 15).

### (11) Effect of CBPexo on fibroblast migration and proliferation

To confirm their regeneration effect during wound healing, CBPexo was labeled using a red-fluorescent dye (PKH26) and incubated with green-fluorescent dye-labeled (PKH67) HSFs. Subsequently, the fluorescence microscopy of PKH26-labeled CBPexo distributed around the PKH67-labeled HSF was performed (FIG. 14A). These fluorescence-labeled exosomes were distributed around green-labeled HSF cells 12 hours after incubation (FIG. 16A). Red-labeled CBPexo significantly migrated to HSFs during incubation for 0 to 24 hours (FIG. 16B).

The CBPexo effect on cell proliferation was confirmed by scratch assay and CFSE proliferation assay. As shown in FIG. 15, it was demonstrated that CBPexo migrate *in vitro* and internalized in fibroblasts to promote proliferation. Furthermore, it was observed that these exosomes are essential mediators of a plasma function and can be used as a novel therapeutic nanoparticle delivery system for wound healing.

### (12) Wound healing effect of CBPexo

Measurement was performed to confirm whether CBPexo, which is the requirement condition for exosome protein delivery, can be internalized in HSFs. CBPexo was incubated with HSFs for 7 days until reaching confluence. Subsequently, the functional effects of CBPexo and ABPexo on fibroblast migration for wound healing were tested. To evaluate whether CBPexo affects fibroblast migration, scratch wound assay was performed. To this end, a wound area containing fibroblasts was scratched, and these fibroblasts were cultured for 24 hours with or without CBPexo, followed by evaluating the wound area. The analysis results indicated that CBPexo significantly increased the migration of fibroblasts compared to the controls and the ABPexo group (FIG. 17A).

Moreover, whether CBPexo increases fibroblast migration in the course of the treatment period was statistically analyzed (FIG. 17B).

As the result of carboxyfluorescein succinimidyl ester (CFSE) assay, the proliferative response of fibroblast when treated with CBPexo or ABPexo was similar to that observed in the wound-closure assay (FIG. 15C). In addition, to determine the main mechanism during migration stimulation and HSF proliferation, a test was performed after HSFs were pre-treated with MMC, which was used as an antiproliferative agent. As a result, it was confirmed that even when the proliferation of fibroblasts was inhibited by MMC, the proliferation increases in the CBPexo-added group, compared to the ABPexo-treated other controls (FIG. 17C). Moreover, in the CBPexo-added group, although the proliferation ability was inhibited by MMC, apoptosis was reduced compared to the control groups (FIG. 17D).

These results demonstrated that CBPexo can promote HSF migration stimulation and fibroblast proliferation during wound healing. Fibroblasts are the major effector cells responsible for soft tissue wound healing, and their proliferation and migration are essential for wound healing, wound contraction, collagen synthesis, and tissue remodeling. This result showed that these CBPexo migrates and is internalized in the fibroblasts, and thus CBPexo can significantly improve the proliferation and migration of fibroblasts compared to ABPexo.

### (13) Enhanced endothelial cell tube formation and polarization of M2 Mϕ by CBPexo

During secondary wound healing known as a proliferation phase, alternatively activated M2 Mϕ was involved in the proliferation and migration of fibroblasts and angiogenesis, thereby reducing inflammation and repairing wounds.

FIG. 18A shows the representative image of HUVEC tube formation for each group. Therefore, the ability of CBPexo to upregulates angiogenesis was evaluated. CBPexo increased the numbers of colony-forming unit-granulocytes, -erythrocytes, - monocytes, and -megakaryocytes (CFU-GEMMs); and macrophage differentiationrelated CFU granulocytes, -monocytes (CFU-GMs), and CFU-monocytes (CFU-Ms) (FIG. 18B). CBPexo promoted macrophage proliferation and induced differentiation of a pro-inflammatory M1 phenotype into anti-inflammatory M2 phenotype. When CBPexo was added and incubated with classically activated M1 Mϕ, CD80 and CD86, IL-12 expression was highly downregulated, whereas CD163, CD206, and IL-10 expression was upregulated (FIG. 18C). These results suggest that classically activated M1 Mϕ was successfully polarized to M2 Mϕ through CBPexo-induced phenotypic switching.

### <Example 2> Preparation and immunosuppressive effect of artificial exosomes (exosome mimetics)

As a result of the proteomic analysis of CBPexo, it was confirmed that exosome proteins (PIP, HSP72, GAL-7, GAL-3 and S100-A7) are associated with immunoregulation and wound healing. MICA induces natural killer cell proliferation, and MHC-I deficiency jeopardizes immune activation. In addition, MHC and MICA/B induce endothelial cell proliferation and affect the wound healing effect. Therefore, by silencing MHC and MICA/B using the CRISPR/cas9 system, cells without immune-activating effect may be generated. HEK 293T cells include high transfection efficiency and homologous haploids of HLA class I (A*02:01, B*07:02 and C*07:02) and MICA/B, and thus have some advantages. For the complete removal of HLA class I and MICA/B genes, 7 types of plasmids encoding a Cas9 protein and gRNA targeting exon 2 and 3 of HLAA-A, HLA-B, HLA-C and MICA/B genes were used (Hong, C.H., et al., J Immunother, 2017. 40(6): p. 201-210). These H1ME-5 cells exerting the deficiency of the HLA class I and MICA/B expression were analyzed by flow cytometry (FIG. 19), and transduced by lentivirus encoding GAL-3, MMP-9, HSP72, S100A7, GAL-7, and PIP genes. Six days after transduction, GAL-3, MMP-9, HSP72, S100A7, GAL-7, and PIP-positive cells were sorted.

The size distribution of exosomes released from H1ME-5 was characterized using DLS, and the particle size of the observed exosomes was 145.61±75.89 nm (FIG. 19B).

Since H1ME-5-derived exosomes have no immunosuppressive effect, the H1ME-5 cells are stable in producing CBPexo mimetics. In addition, the H1ME-5 cells are suitable for producing artificial exosomes because exosomes derived from the H1ME-5 cells have no wound healing effect (FIGS. 19C and19D).

90% or more of the H1ME-5 cells expressed GAL-3, MMP-9, HSP72, S100A7, GAL-7 and PIP, assessed through flow cytometry (FIG. 19E). In addition, the exosomes derived from this cell line expressed target molecules such as GAL-3, MMP-9, HSP72, S100A7, GAL-7 and PIP (FIG. 19F).

Among various CBPexo mimetics, the exosomes expressing MMP-9 and HSP72 exhibited significant immunosuppressive effects, and when the exosomes expressing MMP-9 and HSP72 were mixed at half the concentration and introduced, the synergistic effect of immunosuppression was exhibited (FIGS. 19G and 19H). HSP72 increases MMP-9 production. Therefore, these data indicate that, among various immunosuppressive molecules present in CBPexo, MMP-9 plays a pivotal role.

### <Example 3> Wound healing effect of artificial exosomes (exosome mimetics)

The previous proteomic analysis result for CBPexo is summarized in Table 2. The differential expression levels of the proteins in CBPexo were highlighted in color in Table 2. Red is a CBPexo protein that significantly affects wound healing, and blue is a protein having no wound healing effect.

To confirm the PIP, HSP72, GAL-7, GAL-3, and S100-A7 roles, HSFs were stimulated by artificial exosomes expressing a specific target protein. To evaluate the effects of their proteins on fibroblast proliferation, a series of *in vitro* functional analyses were performed by treating these HSFs with the same concentration of CBPexo or artificial exosomes. According to the method of Example 1, the effect of CBPeox or artificial exosomes on HSF migration was evaluated by scratch wound assay. HSP72-exo and PIP-exo significantly improved HSF migrations as assessed by the migration area (FIG. 20A). Among target proteins, only PIPexo promoted fibroblast proliferation (FIG. 20B). The exosomes expressing GAL-7, GAL-3 and S 100-A7 showed neither wound healing effect nor fibroblast proliferation.

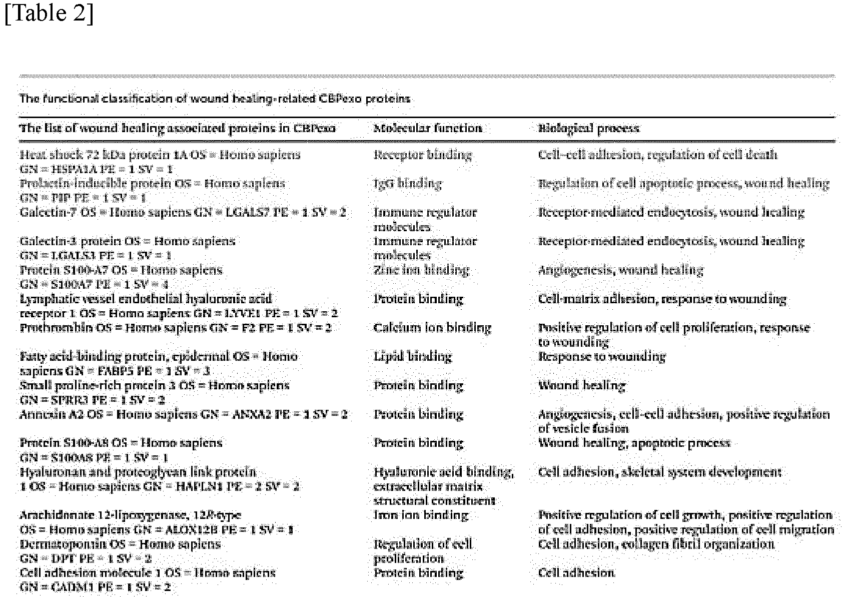

### <Example 4> Effect of exosomes simultaneously expressing HSP72 and PIP on fibroblast proliferation and migration

According to the method of Example 1, 6 days after transduction, cells expressing PIP and HSP72 were simultaneously selected and cultured using a MoFlo XDP cell sorter (FIG. 21A). When HSP72-exo and PIP-exo were added together, the wound healing effect was similar to that of CBPexo and higher than that of exosomes expressing only PIP or HSP72. These exosomes showed a wound healing effect in a concentration-dependent manner (FIG. 21B). Furthermore, the exosomes simultaneously expressing HSP72 and PIP showed a better wound healing effect than those observed when HSP72-exo and PIP-exo are added simultaneously (FIG. 21C). Fibroblasts were labeled with CFSE to be used as a control, and were cultured for 24 hours. After culturing, the cells were harvested and analyzed by flow cytometry. Cell division was identified by sequentially halving CFSE fluorescence with equally spaced peaks on a logarithmic scale. These peaks indicated division cycle numbers. Similar wound-closure results were obtained using a CFSE method, confirming the proliferation rate of normal HSFs with various types of artificial exosomes (FIG. 21D).

Exosomes expressing HSP72 and PIP were generated, and added together or in a concentration-dependent manner to confirm their wound healing effects. As a result, HSP72 and PIP were the most effective proteins in promoting wound healing (among those expressed by CBPexo).

### <Example 5> Confirmation of function of HSP72/PIP in artificial exosomes

Exosomes contain various signaling molecules representing their parent cells. To study the polarization factors in different exosome compositions including a protein, a nucleic acid, a lipid and a metabolite, the study was focused on the CBPexo cargo protein present in an abundant content. In addition, to measure the effect of artificial exosomes on human umbilical vein endothelial cells (HUVECs), a tube formation assay was performed by the method of Example 1. First, compared to those cultured without exosomes, HSP72-exo and PIP-exo increased a node number and the total tube length in the network structure, thereby exhibiting the pro-angiogenesis ability of artificial exosomes. The representative image of the HUVEC tube formation for each group is shown in FIG. 22A. Further analysis revealed that HUVECs cultured with HSP72/PIPexo exhibited better angiogenesis ability compared to those cultured with other exosomes (FIG. 22A). In addition, it was confirmed that, among the different types of exosome components, the differentiation of M1 Mϕ into M2 Mϕ was promoted by HSP72/PIP proteins (FIG. 22B).

From the above results, artificial exosomes expressing HSP72 and PIP can improve wound healing and recovery by improving angiogenesis. The present invention first proved that artificial exosomes expressing HSP72 and PIP can promote cutaneous wound recovery by inducing in situ direct conversions of classically activated M1 Mϕ into polarized M2-like phenotypes.

### [Industrial Applicability]

The present invention can be applied in the field of pharmaceuticals for autoimmune disease or wound healing.

## Claims

1. An immunosuppressive or wound healing composition, comprising:
cord blood plasma-derived exosomes in which the expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) is higher than that of adult plasma-derived exosomes as an active ingredient.

2. The composition of claim 1, wherein the immunosuppression is for alleviating, preventing or treating an immune disease selected from a graft-versus-host disease, an autoimmune disease, a hyperproliferative skin disease, a chronic obstructive pulmonary disease (COPD), allergic asthma, bronchitis, allergic rhinitis, and autoimmune hepatitis.

3. The composition of claim 1, wherein the wound healing is for alleviating or treating a disease selected from wound healing, atopic dermatitis, systemic sclerosis, and myocardial infarction.

4. An immunosuppression or wound healing method, comprising:
administering an effective amount of cord blood plasma-derived exosomes in which the expression of a gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B 12 (SERPINB 12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) is higher than that of adult plasma-derived exosomes into a subject.

5. The method of claim 4, wherein the immunosuppression is for alleviating, preventing or treating an immune disease selected from a graft-versus-host disease, an autoimmune disease, a hyperproliferative skin disease, a chronic obstructive pulmonary disease (COPD), allergic asthma, bronchitis, allergic rhinitis, and autoimmune hepatitis.

6. The method of claim 4, wherein the wound healing is for alleviating or treating a disease selected from wound healing, atopic dermatitis, systemic sclerosis, and myocardial infarction.

7. A medium composition for inhibiting the differentiation of Th1 and Th17 cells, comprising:
cord blood plasma-derived exosomes in which the expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) is higher than that of adult plasma-derived exosomes as an active ingredient.

8. A method of inhibiting the differentiation of Th1 and Th17 cells *in vitro,* comprising:
culturing cord blood plasma-derived exosomes with naive CD4+T cells *in vitro,* wherein the cord blood plasma-derived exosomes have higher expression of the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) than that of adult plasma-derived exosomes.

9. A cord blood plasma exosome mimetic, which is derived from a HLA and MIC-null cell line and expresses one or more selected from the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1).

10. The exosome mimetic of claim 9, wherein the cord blood plasma exosome mimetic expresses the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1).

11. The exosome mimetic of claim 9, which is separated and isolated from a HLA and MIC-null cell line, after the introduction of a nucleic acid encoding the gene group consisting of galectin-3, matrix metalloproteinase (MMP-9), heat shock protein 72 (HSP72), prolactin-inducible protein (PIP), protein S100-A7 (S100A7), galectin-7 (GAL-7), lysosome-associated membrane glycoprotein 1 (LAMP1), serpin B12 (SERPINB12), lactotransferrin (LTF), alpha-1-acid glycoprotein (ORM1), CD5 antigen-like (CD5L), complement C4-B (C4B), mannan-binding lectin serine protease 1 (MASP1), proteasome subunit alpha type-6 (PSMA6), peroxiredoxin-1 (PRDX1), neutrophil defensin 3 (DEFA3), CD44 antigen and arginase-1 (ARG1) into the HLA and MIC-null cell line.

12. The exosome mimetic of claim 11, wherein the HLA and MIC-null cell line is H1ME-5 (Accession No: KCTC 13602BP).

13. The exosome mimetic of claim 9, which expresses one or more selected from the group consisting of heat shock protein 72 (HSP72) and prolactin-inducible protein (PIP).

14. An immunosuppressive or wound healing composition, comprising the cord blood plasma exosome mimetic of claim 9.

15. The composition of claim 14, wherein the immunosuppression is for alleviating, preventing or treating an immune disease selected from a graft-versus-host disease, an autoimmune disease, a hyperproliferative skin disease, a chronic obstructive pulmonary disease (COPD), allergic asthma, bronchitis, allergic rhinitis, and autoimmune hepatitis.

16. An immunosuppression or wound healing method, comprising:
administering an effective amount of the cord blood plasma exosome mimetic of claim 9 into a subject.

17. The method of claim 16, wherein the immunosuppression is for alleviating, preventing or treating an immune disease selected from a graft-versus-host disease, an autoimmune disease, a hyperproliferative skin disease, a chronic obstructive pulmonary disease (COPD), allergic asthma, bronchitis, allergic rhinitis, and autoimmune hepatitis.

18. The method of claim 16, wherein the wound healing is for alleviating or treating a disease selected from wound healing, atopic dermatitis, systemic sclerosis, and myocardial infarction.
